(19) Europäisches Patentamt
European Patent Office
Office européen des brevets

(11) Veröffentlichungsnummer: **0 006 541**
**A2**

(12) **EUROPÄISCHE PATENTANMELDUNG**

(21) Anmeldenummer: **79101942.5**

(51) Int. Cl.$^3$: **A 01 N 9/20**, A 01 N 9/22

(22) Anmeldetag: **15.06.79**

(30) Priorität: **28.06.78 DE 2828303**

(43) Veröffentlichungstag der Anmeldung: **09.01.80**
**Patentblatt 80/1**

(84) Benannte Vertragsstaaten: **AT BE CH DE FR GB IT NL**

(71) Anmelder: **BAYER Aktiengesellschaft, Zentralbereich Patente,Marken und Lizenzen Bayerwerk, D-5090 Leverkusen 1 (DE)**

(72) Erfinder: **Schmidt, Robert Rudolf, Dr., Hahnenweg 5, D-5000 Köln 80 (DE)**
Erfinder: **Faust, Wilfried, Dr., Elfgenstrasse 16, D-5068 Odenthal 3 (DE)**

(54) **Verwendung von N,N-Diallyl-dichloracetamid zur Verbesserung der Kulturpflanzen-Verträglichkeit von herbizid wirksamen Acetaniliden.**

(57) Das bekannte N,N-Diallyl-dichloracetamid der Formel

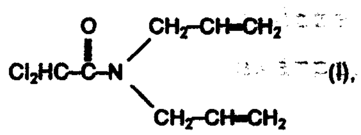

(I),

eignet sich als Gegenmittel («Antidot», «Safener») zum Schutz von Kulturpflanzen vor Schädigungen durch herbizid wirksame Acetanilide — der Formel

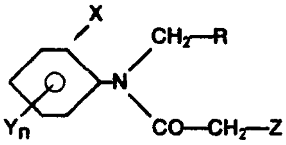

(II)

in welcher

R für einen gegebenenfalls substituierten N-haltigen heterocyclischen Rest steht,

X und Y gleich oder verschieden sind und für Alkyl stehen,

Z für Halogen steht, und

n für 0, 1 oder 2 steht,

sowie deren herbizid wirksamen Säureadditionssalzen und Metallsalz-Komplexen,

— bzw. der Formel

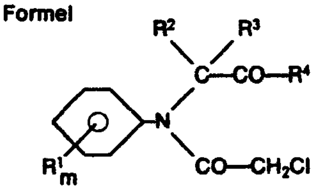

(III)

in welcher

$R^1$ für Alkyl, Halogen, Halogenalkyl, Alkylthio, Alkylsulfonyl, Aminosulfonyl, Cyano oder Nitro steht,

$R^2$ und $R^3$ gleich oder verschieden sind und für Wasserstoff, Alkyl, Halogen, Halogenalkyl oder gegebenenfalls substituiertes Phenyl steht,

$R^4$ für Alkyl oder gegebenenfalls substituiertes Phenyl steht, und

m für ganze Zahlen von 0 bis 5 steht,

— oder der Formel

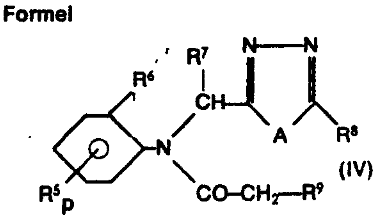

(IV)

in welcher

EP 0 006 541 A2

A für Sauerstoff, Schwefel oder die Gruppierung $>NR^{10}$ steht,

$R^7$ für Wasserstoff oder Alkyl steht,

$R^8$ für Wasserstoff, Alkyl, Halogenalkyl, Alkenyl, Alkinyl, Cycloalkyl, Halogen, gegebenenfalls substituiertes Aryl und Aralkyl oder die Gruppierung $-OR^{11}$, $-SR^{11}$ und $NR^{10}R^{11}$ steht,

$R^{10}$ für Wasserstoff, Alkyl, oder gegebenenfalls substituiertes Aryl steht,

$R^{11}$ für Wasserstoff, Alkyl, Halogenalkyl, Alkenyl, Alkinyl, Cycloalkyl oder gegebenenfalls substituiertes Aralkyl steht,

$R^5$ für Alkyl steht,

$R^6$ für Alkyl oder Halogen steht,

$R^9$ für Halogen steht, und

p für die Zahlen 0, 1 oder 2 steht.

0006541

BAYER AKTIENGESELLSCHAFT
Zentralbereich
Patente, Marken und Lizenzen

5090 Leverkusen, Bayerwerk
Dü/by
IIb

Verwendung von N,N-Diallyl-dichloracetamid zur Verbesserung der Kulturpflanzen-Verträglichkeit von herbizid wirksamen Acetaniliden

Die vorliegende Erfindung betrifft die Verwendung des bekannten N,N-Diallyl-dichloracetamids als Gegenmittel zur Verbesserung der Kulturpflanzen-Verträglichkeit von bestimmten herbizid wirksamen Acetaniliden. Ferner betrifft die vorliegende Erfindung neue Wirkstoffkombinationen, die aus dem bekannten N,N-Diallyldichloracetamid und bestimmten Acetaniliden bestehen und besonders gute selektive herbizide Eigenschaften besitzen.

Unter "Gegenmitteln" ("Safener", "Antidote") sind im vorliegenden Zusammenhang Stoffe zu verstehen, welche befähigt sind, schädigende Wirkungen von Herbiziden auf Kulturpflanzen spezifisch zu antagonisieren, d.h. die Kulturpflanzen zu schützen, ohne dabei die Herbizidwirkung auf die zu bekämpfenden Unkräuter merklich zu beeinflussen.

Es ist bekannt, daß zahlreiche herbizid wirksame Acetanilide beim Einsatz zur Unkrautbekämpfung in Mais und anderen Kulturen mehr oder weniger starke Schäden an den Kulturpflanzen hervorrufen.

Weiterhin ist bekannt, daß N,N-Diallyl-dichloracetamid geeignet ist, um Schädigungen durch herbizide Wirkstoffe, insbesondere Thiolcarbamate, an Kulturpflanzen zu vermindern (vgl. DE-OS 2 218 097). Die Anwendbarkeit dieses Stoffes als Gegenmittel ist jedoch in gewissem Maße abhängig von dem jeweiligen herbiziden Wirkstoff.

Es wurde jetzt gefunden, daß das bekannte N,N-Diallyl-dichloracetamid der Formel

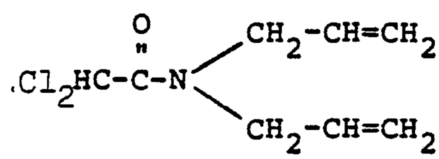

(I)

hervorragend geeignet ist als Gegenmittel zur Verbesserung der Kulturpflanzen-Verträglichkeit von herbizid wirksamen Acetaniliden.

- der Formel

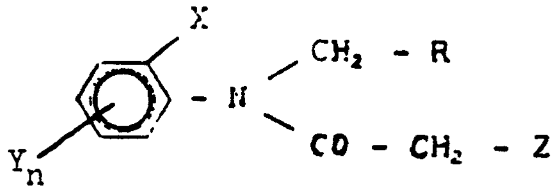

(II)

in welcher

R für einen gegebenenfalls substituierten N-haltigen heterocyclischen Rest steht,

X und Y gleich oder verschieden sind und für Alkyl stehen,

Z für Halogen steht und

n für O, 1 oder 2 steht,

sowie deren herbizid wirksamen Säureadditionssalzen und Metallsalz-Komplexen,

- bzw. der Formel

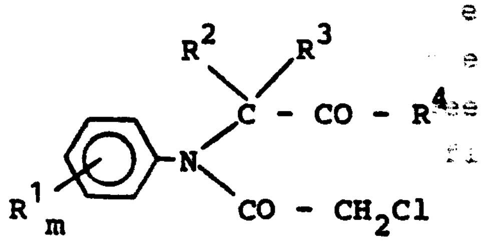

(III)

in welcher

$R^1$ für Alkyl, Halogen, Halogenalkyl, Alkylthio, Alkylsulfonyl, Aminosulfonyl, Cyano oder Nitro steht,

$R^2$ und $R^3$ gleich oder verschieden sind und für Wasserstoff, Alkyl, Halogen, Halogenalkyl oder gegebenenfalls substituiertes Phenyl steht,

$R^4$ für Alkyl oder gegebenenfalls substituiertes Phenyl steht und

m für ganze Zahlen von 0 bis 5 steht,

- oder der Formel

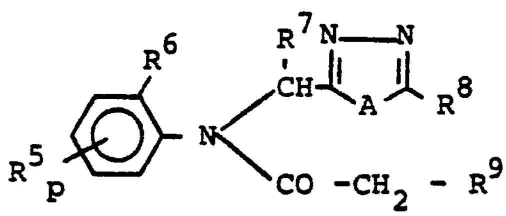

(IV)

in welcher

A für Sauerstoff, Schwefel oder die Gruppierung $>NR^{10}$ steht

$R^7$ für Wasserstoff oder Alkyl steht,

$R^8$ für Wasserstoff, Alkyl, Halogenalkyl, Alkenyl, Alkinyl, Cycloalkyl, Halogen, gegebenenfalls substituiertes Aryl und Aralkyl oder die Gruppierung $-OR^{11}$, $-SR^{11}$ und $NR^{10}R^{11}$ steht,

$R^{10}$ für Wasserstoff, Alkyl, oder gegebenenfalls substituiertes Aryl steht,

$R^{11}$ für Wasserstoff, Alkyl, Halogenalkyl, Alkenyl, Alkinyl, Cycloalkyl oder gegebenenfalls substituiertes Aralkyl steht,

$R^5$ für Alkyl steht,

$R^6$ für Alkyl oder Halogen steht,

$R^9$ für Halogen steht und

p für die Zahlen 0, 1 oder 2 steht.

Weiterhin wurde gefunden, daß die neuen Wirkstoffkombinationen bestehend aus N,N-Diallyl-Dichloracetamid der Formel (I) und mindestens einem Acetanilid der Formel (II), (III) oder (IV) bzw. einem Säureadditionssalz oder Metallsalz-Komplex eines Wirkstoffes der Formel (I) hervorragend geeignet sind zur selektiven Unkrautbekämpfung in Nutzpflanzenkulturen.

Überraschenderweise wird die Kulturpflanzen-Verträglichkeit von herbiziden Wirkstoffen der Formeln (I), (II) und

(IV) bzw. von Säureadditionssalzen oder Metallsalz-Komplexen von Wirkstoffen der Formel (II) durch Mitverwendung von N,N-Diallyl-dichloracetamid der Formel (I) entscheidend verbessert. Unerwartet ist ferner, daß die erfindungsgemäßen Wirkstoffkombinationen aus N,N-Diallyl-dichloracetamid der Formel (I) und Acetaniliden der Formel (II), (III) und/oder (IV) bessere selektive hervizide Eigenschaften besitzen als die betreffenden Wirkstoffe allein.

Das erfindungsgemäß verwendbare N,N-Diallyldichloracetamid der Formel (I) ist bereits bekannt (vgl. DE-OS 2 218 097). Es läßt sich herstellen, indem man Diallylamin mit Dichloracetychlorid gegebenenfalls in Gegenwart eines Säurebindemittels, wie Triäthylamin, Dimethylbenzylamin oder Pyridin, sowie gegebenenfalls eines Verdünnungsmittels, wie z.B. Methylenchlorid oder Acetonitril, bei Temperaturen zwischen 0°C und 40°C umsetzt. Bei dieser Umsetzung kann auch im Überschuß eingesetztes Diallylamin gleichzeitig als Säurebindemittel fungieren. In diesem Fall erübrigt sich die Zugabe eines zusätzlichen Säurebindemittels.

Die praktische Herstellung des erfindungsgemäß verwendbaren N,N-Diallyl-dichloracetamids der Formel (I) geht aus dem nachfolgenden Beispiel hervor:

Beispiel 1

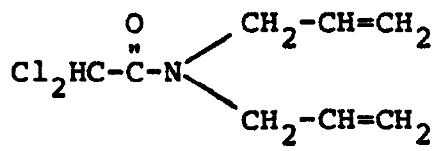

(I)

Eine Lösung von 19,4 g (0,2 Mol) Di-allylamin in 200 ml Acetonitril wird bei Raumtemperatur unter Rühren mit 14,8 g (0,1 Mol) Dichloracetylchlorid versetzt.

Danach wird 2 weitere Stunden bei Raumtemperatur gerührt. Anschließend gibt man das Reaktionsgemisch in Wasser. Das entstehende Gemisch wird mehrfach mit Methylenchlorid extrahiert. Die vereinigten organischen Phasen werden nacheinander mit verdünnter Salzsäure und Wasser gewaschen, über Magnesiumsulfat getrocknet und unter vermindertem Druck eingeengt. Der verbleibende Rückstand wird fraktioniert destilliert. Man erhält auf diese Weise N,N-Diallyl-dichloracetamid.

Das erfindungsgemäß verwendbare N,N-Diallyldichloracetamid der Formel (I) eignet sich, - wie bereits erwähnt -, zur Verbesserung der Kulturpflanzen-Verträglichkeit von herbizid wirksamen Acetaniliden der Formel (II), (III) und (IV) bzw. von Säureadditions-Salzen oder Metallsalz-Komplexen von Wirkstoffen der Formel (II). Als Beispiele für Acetanilide der Formel (II) bzw. davon abgeleitete Säureadditions-Salze seien im einzelnen genannt:

2-Methyl-6-äthyl-N-(pyrazol-1-yl-methyl)-chloracetanilid
2,6-Diäthyl-N-(pyrazol-1-yl-methyl)-chloracetanilid
2,6-Diäthyl-N-(1,2,4-triazol-1-yl-methyl)-chloracetanilid
2,6-Dimethyl-N-(1,2,4-triazol-1-yl-methyl)-chloracetanilid
2-Methyl-N-(pyrazol-1-yl-methyl)-chloracetanilid
2,5-Dimethyl-N-(pyrazol-1-yl-methyl)-chloracetanilid
2,3-Dimethyl-N-(pyrazol-1-yl-methyl)-chloracetanilid
2-Methyl-6-äthyl-N-(pyrazol-1-yl-methyl)-chloracetanilid-hydrochlorid
2,6-Diäthyl-N-(pyrazol-1-yl-methyl)-chloracetanilid-hydrochlorid
2,6-Diäthyl-N-[(3,5-dimethyl-pyrazol-1-yl)-methyl]-chloracetanilid
2,6-Diäthyl-N-[(3-chlor-1,2,4-triazolyl)-methyl]-chloracetanilid
2-Methyl-6-äthyl-N-[(3,5-dimethyl-pyrazol-1-yl)-methyl]-chloracetanilid

2-tert.-Butyl-N-(pyrazol-1-yl-methyl)-chloracetanilid

2-Methyl-6-äthyl-N-[(3-brom-5-methyl-pyrazolyl)-methyl]-chloracetanilid

2-Methyl-6-äthyl-N-[(4-chlor-pyrazol-1-yl)-methyl]-chloracetanilid

2-Methyl-6-äthyl-N-[(3-chlor-1,2,4-triazolyl)-methyl]-chloracetanilid

2,6-Diäthyl-N-[(4-chlor-pyrazol-1-yl)-methyl]-chloracetanilid

Als Beispiele für Acetanilide der Formel (III) seien im einzelnen genannt:

2,6-Dimethyl-N-(benzoyl-methyl)-chloracetanilid

2,6-Dimethyl-N-(4-chlorbenzoyl-methyl)-chloracetanilid

2-Methyl-6-äthyl-N-(benzoyl-methyl)-chloracetanilid

Als Beispiele für Acetanilide der Formel (IV) seien im einzelnen genannt:

2,6-Diäthyl-N-[(2-methyl-1,3,4-oxadiazol-5-yl)-methyl]-chloracetanilid

2,6-Dimethyl-N-[(2-methyl-1,3,4-oxadiazol-5-yl)-methyl]-chloracetanilid

2-Äthyl-6-methyl-N-[(2-methyl-1,3,4-oxadiazol-5-yl)-methyl]-chloracetanilid

2-tert.-Butyl-N-[2-methyl-1,3,4-oxadiazol-5-yl)-methyl]-chloracetanilid

Die herbizid wirksamen Acetanilide der Formel (II) sowie deren Säureadditionssalze und Metallsalz-Komplexe sind bislang noch nicht bekannt. Sie lassen sich jedoch herstellen, indem man

a) N-Halogenmethyl-halogenacetanilide der Formel

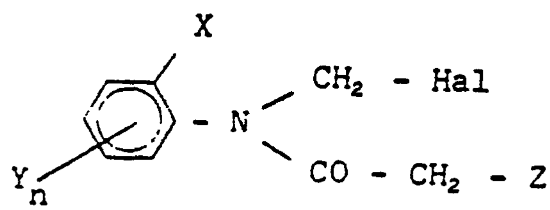

(V)

in welcher

X,Y,Z und n die oben angegebene Bedeutung haben und

Hal für Halogen, insbesondere Chlor oder Brom steht,

mit Heterocyclen der Formel

R-M (VI)

in welcher

R die oben angegebene Bedeutung hat und

M für Wasserstoff oder ein Alkalimetall steht,

gegebenenfalls in Gegenwart eines Verdünnungsmittels und eines Säurebindemittels umsetzt und gegebenenfalls anschließend eine Säure oder ein Metallsalz addiert.

Verwendet man 2,6-Diäthyl-N-chlormethyl-chloracetanilid und Pyrazol als Ausgangsstoffe, so kann der Reaktionsablauf des Verfahrens (a) durch das folgende Formelschema wiedergegeben werden:

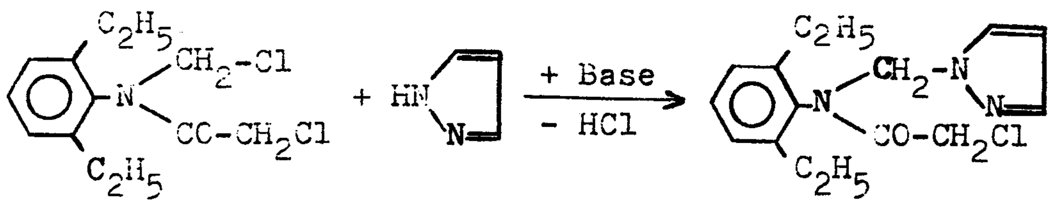


Die bei dem Verfahren (a) als Ausgangsstoffe zu verwendenden N-Halogenmethyl-halogenacetanilide sind durch die Formel (V) allgemein definiert. In dieser Formel sind X und Y gleich oder verschieden und stehen vorzugsweise für geradkettiges oder verzweigtes Alkyl mit 1 bis 4 Kohlenstoffatomen. Z steht vorzugsweise für die Halogene Chlor oder Brom und der Index n hat die oben angegebene Bedeutung.

Die N-Halogenmethyl-halogenacetanilide der Formel (V) sind bekannt oder lassen sich nach bekannten Methoden herstellen (vgl. US-Patentschriften 3 630 716 und 3 637 847). Man erhält sie z.B., indem man entsprechende Aniline mit Paraformaldehyd in Gegenwart katalytischer Mengen Kaliumhydroxid umsetzt und die entstehenden Phenylazomethine mit einem Halogenacetylhalogenid, beispielsweise Chloracetylchlorid, versetzt.

Die N-Halogenmethyl-halogenacetanilide der Formel (V) können auch nach einem neuen Verfahren erhalten werden, indem man bekannte Halogenacetanilide der Formel

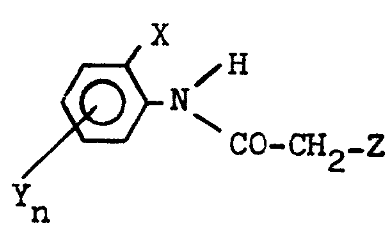

(VII)

in welcher
X,Y,Z und n die oben angegebene Bedeutung haben,

mit mindestens 1 Mol Formaldehyd oder Formaldehyd abgebenden Stoffen, beispielsweise Paraformaldehyd, und einem Halogenierungsmittel, wie einer Halogenwasserstoffsäure oder einem anorganischen bzw. organischen Säurehalogenid, sowie einem wasserbindenden Mittel, beispielsweise Natriumsulfat, in an sich bekannter Weise bei Temperaturen zwischen -10°C und 150°C, vorzugsweise zwischen 10 und 70°C, gegebenenfalls in Gegenwart eines inerten organischen Lösungsmittels, beispielsweise Toluol, umsetzt (vgl. Deutsche Offenlegungsschriften 2 119 518 und 2 210 603). Bei Verwendung von anorganischen Säurehalogeniden, wie beispielsweise Thionylchlorid, kann auf die Verwendung eines speziellen Wasserbinders verzichtet werden (vgl. auch Herstellungsbeispiele).

Die außerdem als Ausgangsstoffe zu verwendenden Heterocyclen sind durch die Formel (VI) allgemein definiert. In dieser Formel steht R vorzugsweise für die gegebenenfalls substituierten Azolylreste Pyrazol-1-yl, Imidazol-1-yl, 1,2,4-Triazol-1-yl; 1,2,3-Triazol-1-yl; 1,3,4-Triazol-1-yl und 1,2,3,4-Tetrazol-1-yl sowie für gegebenenfalls substituiertes Pyrrol-1-yl. Als Substituenten kommen vorzugsweise in Frage: Halogen, insbesondere Fluor, Chlor und Brom, sowie Alkyl mit 1 bis 4 Kohlenstoffatomen. M steht vorzugsweise für Wasserstoff und die Alkalimetalle Natrium und Kalium.

Die Heterocyclen der Formel (VI) sind allgemein bekannte Verbindungen der organischen Chemie.

Für die Umsetzung nach dem Verfahren (a) kommen als Verdünnungsmittel vorzugsweise inerte organische Lösungsmittel in Frage. Hierzu gehören vorzugsweise Ketone, wie Diäthylketon, insbesondere Methylisobutylketon; Nitrile, wie Propionitril, insbesondere Acetonitril; Äther, wie Tetrahydrofuran oder Dioxan; aliphatische und aromatische Kohlenwasserstoffe, wie Petroläther, Benzol, Toluol oder Xylol, halogenierte Kohlenwasserstoffe, wie Methylenchlorid, Tetrachlorkohlenstoff, Chloroform oder Chlorbenzol; Ester, wie Essigester; und Formamide, wie insbesondere Dimethylformamid.

Als Säurebindemittel können bei dem Verfahren (a) alle üblicherweise verwendbaren anorganischen und organischen Säureakzeptoren eingesetzt werden. Hierzu gehören vorzugsweise Alkalicarbonate, beispielsweise Natriumcarbonat, Kaliumcarbonat und Natriumhydrogencarbonat, ferner niedere tertiäre Alkylamine, Aralkylamine, aromatische Amine oder Cycloalkylamine, wie z.B. Triäthylamin, Dimethylbenzylamin, Pyridin und Diazabicyclooctan. Es ist auch möglich, einen entsprechenden Überschuß an Azol zu verwenden, worunter im vorliegenden Fall eine Verbindung der Formel (VI) zu verstehen ist.

Die Reaktionstemperaturen können beim Verfahren (a) in einem größeren Bereich variiert werden. Im allgemeinen arbeitet man zwischen etwa 0 und 120°C, vorzugsweise zwischen 20 und 80°C.

Bei der Durchführung des Verfahrens (a) setzt man auf 1 Mol der Verbindungen der Formel (V) vorzugsweise

1 bis 2 Mol des Heterocyclen der Formel (VI) und 1 Mol Säurebinder ein. Zur Isolierung der Verbindungen der Formel (II) wird das Reaktionsgemisch filtriert, das Filtrat mit Wasser gewaschen, getrocknet und eingeengt. Der Rückstand wird gegebenenfalls durch fraktionierte Kristallisation oder Destillation gereinigt.

In einer besonderen Aufarbeitungsform kühlt man das Reaktionsgemisch auf ca. 0°C ab, filtriert und leitet in das Filtrat bei 5 bis -15°C Chlorwasserstoff ein. Die ausfallenden Chloridsalze werden abgesaugt, mit einem organischen Solvens, beispielsweise Essigester, gewaschen und in einem Gemisch aus einem organischen Lösungsmittel, beispielsweise Essigester und Wasser von einem pH-Wert um 12 verteilt. Man trennt die organische Phase ab und isoliert die Verbindungen der Formel (II) in üblicher Weise.

Zur Herstellung von Säureadditionssalzen der Verbindungen der Formel (II) kommen alle Säuren in Frage, die zu physiologisch verträglichen Salzen führen. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Napthalindisulfonsäure.

Die Salze der Verbindungen der Formel (IV) können in einfacher Weise nach üblichen Salzbildungsmethoden, z.B. durch Lösen einer Verbindung der Formel (IV) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (II) kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien. Als Anionen der Salze kommen solche in Betracht, die sich von physiologisch verträglichen Säuren ableiten, welche zu physiologisch verträglichen Salzen führen. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure.

Die Metallsalzkomplexe der Verbindungen der Formel (II) können in einfacher Weise nach üblichen Verfahren erhalten werden, so z.B. durch Lösen des Metallsalzes in Alkohol, z.B. Äthanol, und Hinzufügen zur Verbindung der Formel (II). Man kann Metallsalzkomplexe in bekannter Weise, z.B. durch Abfiltrieren, isolieren und gegebenenfalls durch Umkristallisation reinigen.

Die praktische Herstellung von Acetaniliden der Formel (IV) geht aus den folgenden Beispielen hervor.

Beispiel 2

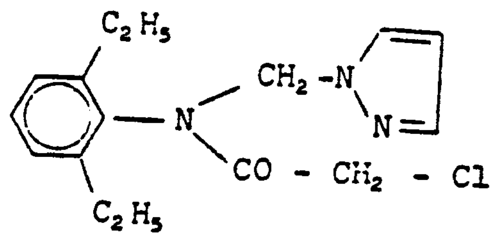

Zu 274,2 g (1 Mol) 2,6-Diäthyl-N-chlormethyl-chloracetanilid in 250 ml wasserfreiem Essigester gibt man unter Rühren eine Mischung aus 68 g (1 Mol) Pyrazol und 106 g (1,05 Mol) Triäthylamin in 150 ml wasserfreiem Essigester, wobei die Temperatur auf 30°C ansteigt. Man rührt 1 Stunde bei Raumtemperatur nach. Für die Aufarbeitung ergeben sich zwei Möglichkeiten:

1) Das Reaktionsgemisch wird filtriert, das Filtrat mit Wasser neutral gewaschen, über Natriumsulfat getrocknet und im Vakuum eingedampft. Nach einer fraktionierten Kristallisation mit Ligroin erhält man 171,2 g (56 % der Theorie) 2,6-Diäthyl-N-(pyrazol-1-yl-methyl)-chloracetanilid vom Schmelzpunkt 67°C in Form farbloser Kristalle.

2) Das Reaktionsgemisch wird auf 0°C abgekühlt, filtriert und der Filterrückstand mit 10 ml kaltem Essigester nachgewaschen. In das Filtrat werden bei 0 bis -10°C 50 g (1,4 Mol) trockener Chlorwasserstoff eingeleitet. Man saugt anschließend die ausgefallenen Hydrochlorid-Salze ab, wäscht mit 50 ml kaltem Essigester nach und verteilt den festen Rückstand zwischen 0,5 l Essigester

und 0,5 l wäßriger Natriumhydroxid-Lösung mit einem pH-Wert von 12. Die organische Phase wird abgetrennt, zweimal mit je 0,5 l Natriumchloridlösung gewaschen, über Natriumsulfat getrocknet und im Vakuum eingedampft. Der farblose ölige Rückstand wird mit 60 ml Benzin versetzt, wobei er kristallisiert. Man erhält 220,2 g (72 % der Theorie) 2,6-Diäthyl-N-(pyrazol-1-yl-methyl)-chloracetanilid vom Schmelzpunkt 67°C in Form farbloser Kristalle.

In analoger Weise werden die in der nachfolgenden Tabelle aufgeführten Verbindungen hergestellt:

**Tabelle 1**

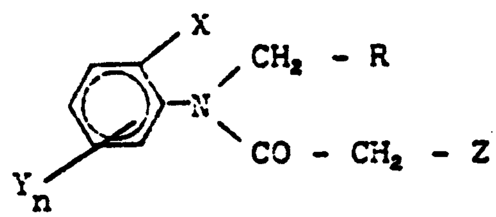

(II)

| Bsp. Nr. | X | Yn | Z | R | Schmelzpunkt (°C) |
|---|---|---|---|---|---|
| 3 | $C_2H_5$ | 6-$C_2H_5$ | Cl | 1,2,4-Triazol-1-yl | 112 |
| 4 | i-$C_3H_7$ | 6-i-$C_3H_7$ | Cl | Pyrazol-1-yl | 134 |
| 5 | $CH_3$ | 6-$C_2H_5$ | Cl | 1,2,4-Triazol-1-yl | 92 |
| 6 | $CH_3$ | 6-$C_2H_5$ | Cl | Pyrazol-1-yl | 57 |
| 7 | $C_2H_5$ | 4,6-$(CH_3)_2$ | Cl | Pyrazol-1-yl | 82 |
| 8 | $CH_3$ | 4,6-$(CH_3)_2$ | Cl | Pyrazol-1-yl | 92 |
| 9 | $C_2H_5$ | 4-$CH_3$, 6-$C_2H_5$ | Cl | Pyrazol-1-yl | 78 |
| 10 | i-$C_3H_7$ | 6-i-$C_3H_7$ | Cl | 1,3,4-Triazol-1-yl | 196 |
| 11 | i-$C_3H_7$ | 6-i-$C_3H_7$ | Cl | 1,2,4-Triazol-1-yl | 138 |

Tabelle 1(Fortsetzung)

| Beispiel Nr. | X | Yn | Z | R | Schmelzpunkt(°C) |
|---|---|---|---|---|---|
| 12 | $C_2H_5$ | $6-C_2H_5$ | Cl | Pyrrol-1-yl | Oel |
| 13 | $i-C_3H_7$ | — | Cl | 1,2,4-Triazol-1-yl | 118 |
| 14 | $CH_3$ | $6-C_2H_5$ | Cl | 1,2,3,4-Tetrazol-1-yl | Oel |
| 15 | $i-C_3H_7$ | — | Cl | Pyrazol-1-yl | Oel |
| 16 | $C_2H_5$ | — | Cl | 1,2,4-Triazol-1-yl | 81 |
| 17 | $CH_3$ | $6-CH_3$ | Cl | Pyrazol-1-yl | 82 |
| 18 | $CH_3$ | $6-CH_3$ | Cl | 1,2,4-Triazol-1-yl | 110 |
| 19 | $CH_3$ | $5-CH_3$ | Cl | 1,2,4-Triazol-1-yl | Oel |
| 20 | $CH_3$ | — | Cl | Pyrazol-1-yl | 56 |
| 21 | $CH_3$ | — | Cl | 1,2,4-Triazol-1-yl | 88 |
| 22 | $CH_3$ | $5-CH_3$ | Cl | Pyrazol-1-yl | Oel |
| 23 | $CH_3$ | $3-CH_3$ | Cl | 1,2,4-Triazol-1-yl | 114 |
| 24 | $CH_3$ | $3-CH_3$ | Cl | Pyrazol-1-yl | 102 |
| 25 | $C_2H_5$ | $6-CH_3$ | Cl | Pyrazol-1-yl (xHCl) | 87 |
| 26 | $C_2H_5$ | $6-C_2H_5$ | Cl | Pyrazol-1-yl (xHCl) | 67 |
| 27 | $C_2H_5$ | $6-C_2H_5$ | Cl | 3,5-Dimethyl-pyrazol-1-yl | 111 |
| 28 | $C_2H_5$ | $6-C_2H_5$ | Cl | Brom-methyl-pyrazolyl | 145 |
| 29 | $C_2H_5$ | $6-C_2H_5$ | Cl | 3-Chlor-1,2,4-triazol-1-yl | 110 |
| 30 | $CH_3$ | $6-C_2H_5$ | Cl | 3,5-Dimethyl-pyrazol-1-yl | 90 |
| 31 | $C_2H_5$ | $6-C_2H_5$ | Cl | 3-Methyl-pyrazol-1-yl | 89 |
| 32 | $C_2H_5$ | $6-CH_3$ | Cl | 3-Methyl-pyrazol-1-yl | 113 |
| 33 | $C(CH_3)_3$ | — | Cl | Pyrazol-1-yl | Oel |
| 34 | $C(CH_3)_3$ | — | Cl | 1,2,4-Triazol-1-yl | 118 |
| 35 | $C_2H_5$ | $6-CH_3$ | Cl | Brom-methyl-pyrazolyl | 80 |

Tabelle 1 (Fortsetzung)

| Beispiel Nr. | X | Yn | Z | R | Schmelzpunkt (°C) |
|---|---|---|---|---|---|
| 36 | $CH_3$ | 6-$C_2H_5$ | Cl | 4-Chlor-pyrazol-1-yl | 91 |
| 37 | $CH_3$ | 6-$C_2H_5$ | Cl | 3-Chlor-1,2,4-triazol-1-yl | 121 |
| 38 | $C_2H_5$ | 6-$CH_3$ | Cl | 2,4,5-Trichlor-imidazol-1-yl | 158 |
| 39 | $C_2H_5$ | 6-$C_2H_5$ | Cl | 4-Chlor-pyrazol-1-yl | 110 |
| 40 | $C_2H_5$ | 6-$C_2H_5$ | Cl | 1,2,3,4-Tetrazol-1-yl | 110 |
| 41 | $C_2H_5$ | 6-$C_2H_5$ | Br | Pyrazol-1-yl | 68 |
| 42 | $CH_3$ | 6-$C_2H_5$ | Br | Pyrazol-1-yl | 67 |
| 43 | $C_2H_5$ | 6-$C_2H_5$ | Cl | Imidazol-1-yl | Oel |
| 44 | $C_2H_5$ | 6-$C_2H_5$ | Br | 1,2,4-Triazol-1-yl | 90 |
| 45 | $CH_3$ | 6-$C_2H_5$ | Br | 1,2,4-Triazol-1-yl | 78 |

Herstellung von Ausgangsprodukten

Beispiel 2 a

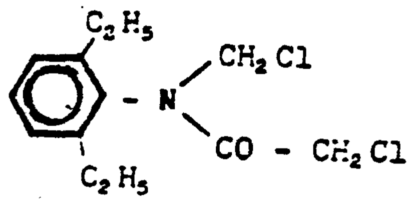

(Variante α)

Zu einer Lösung von 225,7 g (1 Mol) 2,6-Diäthyl-chloracetanilid in 1,5 l Toluol werden 45 g (1,5 Mol) Paraformaldehyd gegeben. Man erwärmt auf 40°C und tropft unter Rühren 179 g (1,5 Mol) Thionylchlorid zu, wobei eine lebhafte Gasentwicklung einsetzt. Man läßt bei 40°C nachrühren, bis die Gasentwicklung beendet ist. Danach wird filtriert und das Filtrat im Vakuum eingeengt. Nach Entgasung des Rückstands im Hochvakuum erhält man 268,7 g (98 % der Theorie) 2,6-Diäthyl-N-chlormethyl-chloracetanilid als farbloses Öl.

(Variante ß)

Zu einer Lösung von 225,7 g (1 Mol) 2,6-Diäthyl-chloracetanilid in 1,5 l wasserfreiem Toluol werden 45 g (1,5 Mol) Paraformaldehyd und 100 g wasserfreies Natriumsulfat gegeben. Unter Rühren und Erwärmung auf 50°C leitet man solange trockenen Chlorwasserstoff ein, bis die milchige Suspension des Paraformaldehyds verschwunden ist. Danach werden nochmals 100 g wasserfreies Natriumsulfat zugegeben, eine Stunde bei 50°C nachgerührt und filtriert. Das Filtrat wird im Vakuum eingeengt. Nach

Entgasung des Rückstands erhält man 263,2 g (96 % der Theorie) 2,6-Diäthyl-chloracetanilid als farbloses Öl.

Analog Beispiel 2a werden die Verbindungen der nachfolgenden Tabelle 2 erhalten.

Tabelle 2

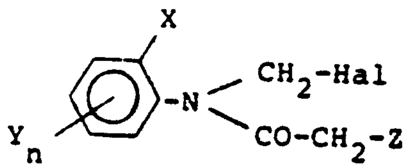

(V)

| Beispiel Nr. | X | $Y_n$ | Z | Hal | Schmelzpunkt bzw. Brechungsindex |
|---|---|---|---|---|---|
| 4 a | $i-C_3H_7$ | $6-i-C_3H_7$ | Cl | Cl | nicht isoliert |
| 5 a | $CH_3$ | $6-C_2H_5$ | Cl | Cl | 91 |
| 7 a | $C_2H_5$ | $4,6-(CH_3)_2$ | Cl | Cl | nicht isoliert |
| 8 a | $CH_3$ | $4,6-(CH_3)_2$ | Cl | Cl | " |
| 9 a | $C_2H_5$ | $4-CH_3$<br>$6-C_2H_5$ | Cl | Cl | " |
| 13 a | $i-C_3H_7$ | - | Cl | Cl | 90 |
| 16 a | $C_2H_5$ | - | Cl | Cl | nicht isoliert |
| 17 a | $CH_3$ | $6-CH_3$ | Cl | Cl | 88 |
| 19 a | $CH_3$ | $5-CH_3$ | Cl | Cl | nicht isoliert |
| 23 a | $CH_3$ | $3-CH_3$ | Cl | Cl | 40 |
| 33 a | $C(CH_3)_3$ | - | Cl | Cl | nicht isoliert |
| 41 a | $C_2H_5$ | $6-C_2H_5$ | Br | Br | " |
| 42 a | $CH_3$ | $6-C_2H_5$ | Br | Br | " |

Die Acetanilide der Formel (II) bzw. deren Säureadditions-Salze und Metallsalz-Komplexe weisen starke herbizide Wirkungen, insbesondere gegen Gräser, auf. Sie können deshalb zur selektiven Unkraut- und insbesondere Ungräserbekämpfung eingesetzt werden.

Die herbizid wirksamen Acetanilide der Formel (III) sind ebenfalls bisher noch nicht bekannt. Sie lassen sich jedoch herstellen, indem man

b) N-Acylmethylaniline der Formel

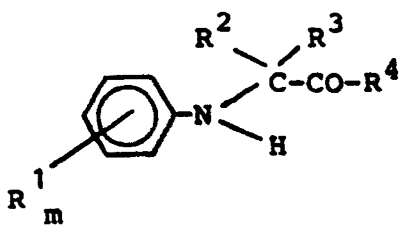

(VIII)

in welcher
$R^1$,$R^2$,$R^3$,$R^4$ und m die oben angegebene Bedeutung haben,

mit Chloracetylchlorid in Gegenwart eines Verdünnungsmittels umsetzt.

Verwendet man 2,6-Dimethyl-N-benzoylmethyl-anilin und Chloracetylchlorid als Ausgangsstoffe, so kann der Reaktionsablauf des Verfahrens (b) durch das folgende Formelschema wiedergegeben werden:

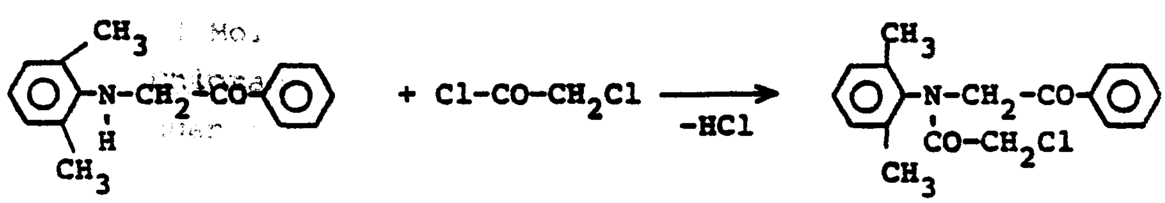

Die bei dem Verfahren (b) als Ausgangsstoffe zu verwendenden N-Acylmethyl-aniline sind durch die Formel (VIII) allgemein definiert. In dieser Formel steht $R^1$ vorzugsweise für geradkettiges oder verzweigtes Alkyl mit 1 bis 6, insbesondere 1 bis 4 Kohlenstoffatomen, Halogen, insbesondere Fluor, Chlor und Brom, Halogenalkyl mit bis zu 3 Kohlenstoff- und bis zu 5 gleichen oder verschiedenen Halogenatomen, wobei als Halogene insbesondere Fluor und Chlor stehen, beispielhaft sei Trifluormethyl genannt; ferner vorzugsweise für Alkylthio und Alkylsulfonyl mit 1 bis 4 Kohlenstoffatomen im Alkylteil sowie für Aminosulfonyl, Cyano und Nitro. $R^2$ und $R^3$ sind gleich oder verschieden und stehen vorzugsweise für Wasserstoff, geradkettiges oder verzweigtes Alkyl mit 1 bis 4 Kohlenstoffatomen, Halogen, insbesondere Fluor, Chlor und Brom, Halogenalkyl mit bis zu 3 Kohlenstoff- und bis zu 5 gleichen oder verschiedneen Halogenatomen, wobei als Halogene insbesondere Fluor und Chlor stehen sowie vorzugsweise für gegebenenfalls einfach oder mehrfach substituiertes Phenyl, wobei als Substituenten vorzugsweise die für $R^1$ genannten Reste in Frage kommen. $R^4$ steht vorzugsweise für geradkettiges oder verzweigtes Alkyl mit 1 bis 6, insbesondere 1 bis 4 Kohlenstoffatomen sowie für gegebenenfalls einfach oder mehrfach substituiertes Phenyl, wobei als Substituenten vorzugsweise die bei $R^1$ bereits genannten Reste sowie Phenyl und Phenoxy, die ebenfalls durch $R^1$ substituiert sein können, in Frage kommen.

Die N-Acylmethyl-aniline der Formel (VIII) sind bekannt (vgl. u.a. Chem.Ber. 25, 2865 (1892) sowie Chem.Soc. 1943, 63) oder lassen sich nach bekannten Methoden

herstellen. Man erhält sie z.B., indem man Aniline mit α-Halogenketonen in Gegenwart eines organischen Lösungsmittels, wie z.B. Äthanol, umsetzt (vgl. auch die Herstellungsbeispiele).

Als Verdünnungsmittel kommen für die Umsetzung nach Verfahren (b) vorzugsweise inerte organische Lösungsmittel in Frage. Hierzu gehören vorzugsweise Ketone, wie Diäthylketon, insbesondere Aceton und Methyläthylketon; Nitrile, wie Propionitril, insbesondere Acetonitril; Aether, wie Tetrahydrofuran oder Dioxan; aliphatische und aromatische Kohlenwasserstoffe, wie Petroläther, Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Die Reaktionstemperaturen können bei der Durchführung des Verfahrens (b) in einem größeren Bereich variiert werden. Im allgemeinen arbeitet man zwischen 0 und 120°C, vorzugsweise zwischen 20 und 100°C.

Bei der Durchführung des Verfahrens (b) setzt man vorzugsweise 1 Mol der Verbindung der Formel (VIII) 1 bis 3 Mol Chloracetylchlorid ein. Die Isolierung der Verbindungen der Formel (III) erfolgt in üblicher Weise.

Die praktische Herstellung von Acetaniliden der Formel (III) geht aus den folgenden Beispielen hervor:

Beispiel 46

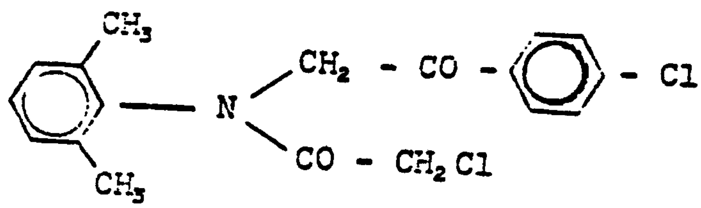

In eine Lösung von 18,5 g (0,068 Mol) 2,6-Dimethyl-N-(4-chlor-benzoylmethyl)-anilin in 150 ml Benzol werden 16 ml (0,2 Mol) Chloracetylchlorid getropft. Danach läßt man 15 Stunden unter Rückfluß rühren und engt durch Abdestillieren des Lösungsmittels und des überschüssigen Chloracetylchlorids im Vakuum ein. Der Rückstand wird mit einem Gemisch Aether/Petroläther (1:3) verrieben, der entstehende kristalline Rückstand abgesaugt und getrocknet. Man erhält 17,7 g (75 % der Theorie) 2,6-Dimethyl-N-(4-chlorbenzoylmethyl)-chloracetanilid vom Schmelzpunkt 128°C.

Herstellung des Ausgangsproduktes

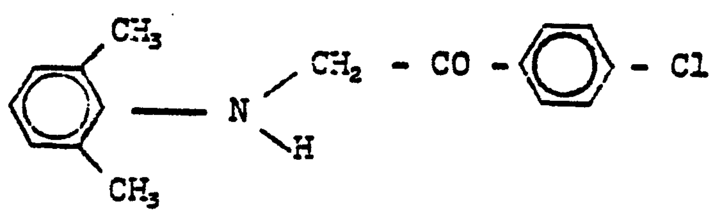

46,7 g (0,2 Mol) ω-Brom-4-chloracetophenon in 40 ml Aethanol werden zu 48,4 g (0,4 Mol) 2,6-Dimethylanilin

in 40 ml Aethanol gegeben und 20 Minuten auf 50°C erwärmt. Danach kühlt man auf 0°C ab, filtriert die entstandenen Kristalle ab und wäscht mit wenig Aethanol nach. Man erhält 30 g (55 % der Theorie) 2,6-Dimethyl-N-(4-chlorbenzoylmethyl)-anilin vom Schmelzpunkt 82°C.

Beispiel 48

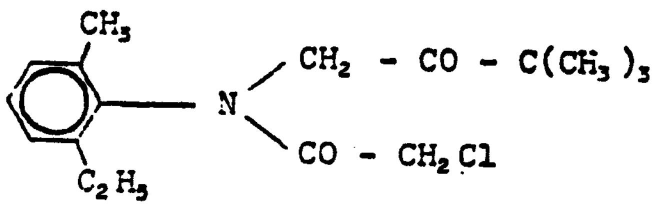

23,3 g (0,1 Mol) 2-Aethyl-6-methyl-N-pivaloylmethyl-anilin werden in 100 ml Benzol gelöst und mit 24 ml (0,3 Mol) Chloracetylchlorid versetzt. Danach läßt man 15 Stunden unter Rückfluß rühren und engt durch Abdestillieren des Lösungsmittels und des überschüssigen Chloracetylchlorids im Vakuum ein. Der ölige Rückstand wird mit Petroläther verrührt, dekantiert, mit Aktivkohle verrührt, filtriert und im Vakuum eingeengt. Der Rückstand wird mit n-Hexan verrührt, der resultierende Feststoff abgesaugt und getrocknet. Man erhält 13,7 g (45 % der Theorie) 2-Aethyl-6-methyl-N-pivaloylmethyl-chloracetanilid vom Schmelzpunkt 86°C.

Herstellung des Ausgangsproduktes

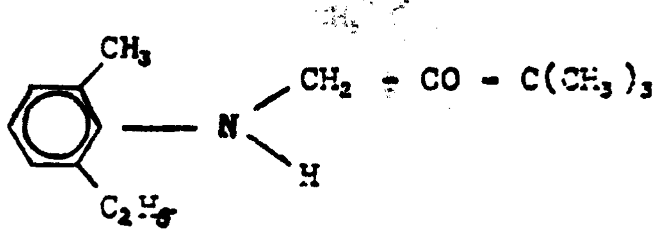

108 g (0,8 Mol) 2-Aethyl-6-methyl-anilin und 53,8 g (0,4 Mol) Monochlorpinakolin werden in 300 ml Toluol 25 Stunden auf 110°C erhitzt. Man läßt abkühlen, filtriert, wäscht das Filtrat mit Wasser, trocknet über Natriumsulfat und engt durch Abdestillieren des Lösungsmittels im Vakuum ein. Der Rückstand wird fraktioniert destilliert. Man erhält 24,1 g (26 % der Theorie) 2-Aethyl-6-methyl-N-pivaloylmethyl-anilin vom Siedepunkt 138 bis 150°C/0,7 mm und einem Brechungsindex von $n_D^{20}$ = 1,5168.

In analoger Weise werden die in der nachfolgenden Tabelle 3 aufgeführten Verbindungen hergestellt.

0006541

**Tabelle: 3**

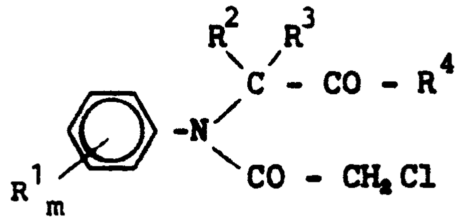

(III)

| Bsp. Nr. | $R^1_m$ | $R^2$ | $R^3$ | $R^4$ | Schmelzpunkt(°C) bzw. Brechungsindex |
|---|---|---|---|---|---|
| 48 | 2-$CH_3$ | H | H | 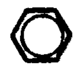 | 138 |
| 49 | 2-$CH_3$ | H | H | 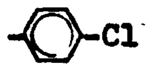 | 140 |
| 50 | 2,6-$(C_2H_5)_2$ | H | H | 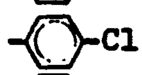 | 134 |
| 51 | 2,6-$(C_2H_5)_2$ | H | H | 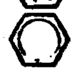 | 116 |
| 52 | 2-Cl | H | H | 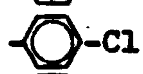 | 124 |
| 53 | 2,6-$(CH_3)_2$ | H | H | 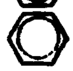 | 100 |
| 54 | 4-Cl | H | H | 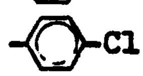 | 114 |
| 55 | 2,6-$(CH_3)_2$ | $CH_3$ | H | $CH_3$ | 104 |
| 56 | 2,6-$(i\text{-}C_3H_7)_2$ | H | H | 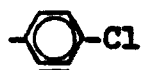 | 200 |
| 57 | 2,6-$(C_2H_5)_2$, 4-$CH_3$ | H | H | 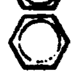 | 112 |
| 58 | 2,6-$(i\text{-}C_3H_7)_2$ | H | H | 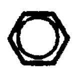 | 140 |
| 59 | 2,6-$(CH_3)_2$ | H | H | 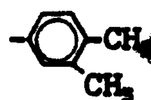 | 90 |
| 60 | 2-$C_2H_5$, 6-$CH_3$ | H | H | 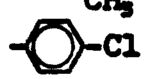 | 70 |
| 61 | 2,6-$(CH_3)_2$ | H | H | 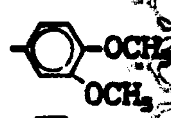 | 114 |
| 62 | 2-$C_2H_5$, 4,6-$(CH_3)_2$ | H | H | 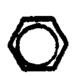 | $n_D^{20}$ =1,5680 |
| 63 | 2,6-$(CH_3)_2$ | H | H | 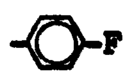 | 104 |
| 64 | 2,4,6-$(CH_3)_3$ | H | H | 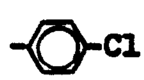 | 134 |

<u>Tabelle 3</u> (Fortsetzung)

| Bsp. Nr. | $R^1_m$ | $R^2$ | $R^3$ | $R^4$ | Schmelzpunkt(°C) bzw Brechungsindex |
|---|---|---|---|---|---|
| 65 | 2,4,6-$(CH_3)_3$ | H | H | 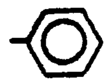 | $n_D^{20}$ = 1,5610 |
| 66 | 2,6-$(CH_3)_2$ | H | 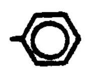 | 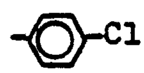 | 149 |
| 67 | 2,6-$(CH_3)_2$ | H | $CH_3$ | 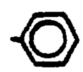 | 84 |

0006541

In analoger Weise können die in der nachfolgenden Tabelle 4 aufgeführten Verbindungen erhalten werden.

**Tabelle 4**

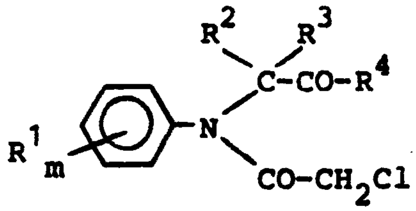

(III)

| Beispiel Nr. | $R^1_m$ | $R^2$ | $R^3$ | $R^4$ |
|---|---|---|---|---|
| 68 | 3,5-$(CF_3)_2$ | H | H | 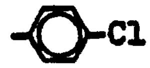 |
| 69 | 2,6-$(CH_3)_2$ | H | H | 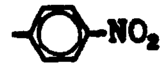 |
| 70 | 2,6-$(CH_3)_2$ | H | H | 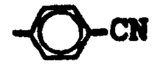 |
| 71 | 2,6-$(CH_3)_2$ | H | H | 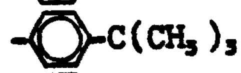 |
| 72 | 2,6-$(CH_3)_2$, 4-$SO_2NH_2$ | H | H | 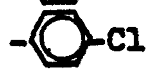 |
| 73 | 2-Cl,6-$CH_3$ | H | H | 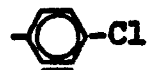 |
| 74 | 2-$C_2H_5$,6-$CH_3$ | $CH_3$ | $CH_3$ | 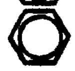 |
| 75 | 2-$C_2H_5$,6-$CH_3$ | $CH_3$ | $CH_3$ | 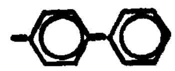 |
| 76 | 2-$C_2H_5$,6-$CH_3$ | $CH_3$ | $CH_3$ | 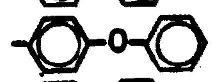 |
| 77 | 2-$C_2H_5$,6-$CH_3$ | $CH_3$ | $CH_3$ | 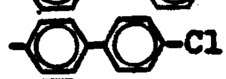 |
| 78 | 2-$C_2H_5$,6-$CH_3$ | $CH_3$ | $CH_3$ | 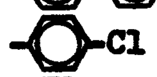 |
| 79 | 2-$C_2H_5$,6-$CH_3$ | H | $CH_3$ | 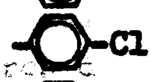 |
| 80 | 2-$C_2H_5$,6-$CH_3$ | H | $CH_3$ | 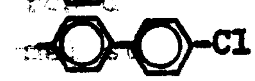 |
| 81 | 2,6-$(CH_3)_2$ | H | 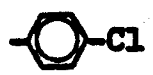 | 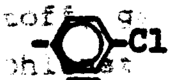 |
| 82 | 2,6-$(CH_3)_2$ | H | 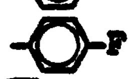 | 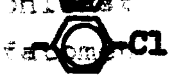 |
| 83 | 2,6-$(CH_3)_2$ | H | 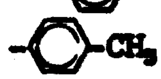 | 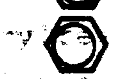 |
| 84 | 2,6-$(CH_3)_2$ | H | 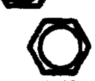 | 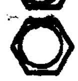 |
| 85 | 2,6-$(CH_3)_2$ | H | 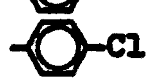 | 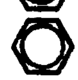 |
| 86 | 2,6-$(CH_3)_2$ | H | $CH_3$ | 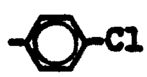 |

Die Acetanilide der Formel (III) besitzen starke herbizide Eigenschaften. Sie eignen sich daher zur Unkrautbekämpfung. Insbesondere lassen sie sich zur selektiven Unkraut- und Ungrasbekämpfung einsetzen.

Die herbizid wirksamen Acetanilide der Formel (IV) sind ebenfalls bisher noch nicht bekannt. Sie lassen sich jedoch herstellen, indem man

c) N-Azolylalkylaniline der Formel

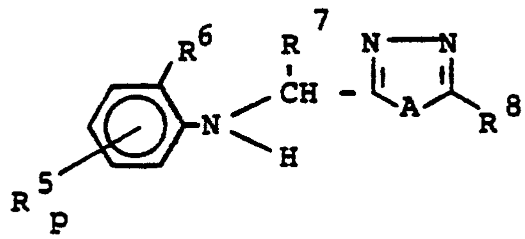

(IX)

in welcher
$R^5$, $R^6$, $R^7$, $R^8$, A und p die oben angegebene Bedeutung haben,

mit Halogenessigsäurechloriden bzw. -anhydriden der Formeln

$R^9-CH_2-CO-Cl$ (Xa)

bzw.

$(R^9-CH_2-CO)_2O$ (Xb)

in welchen
$R^9$ die oben angegebene Bedeutung hat,

in Gegenwart eines Verdünnungsmittels und gegebenenfalls in Gegenwart eines Säurebindemittels umsetzt.

Verwendet man 2,6-Diäthyl-N-(3-methylthio-4-methyl-1,2,4-triazol-5-yl-methyl)-anilin und Chloracetylchlorid als Ausgangsstoffe, so kann der Reaktionsablauf des Verfahrens (c) durch das folgende Formelschema wiedergegeben werden:

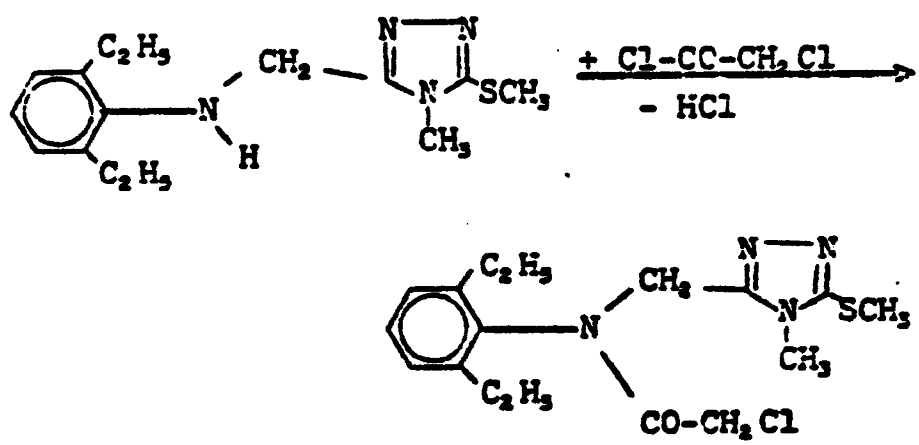


Die bei der Durchführung des Verfahrens (c) als Ausgangsstoffe benötigten N-Azolylalkylaniline sind durch die Formel (IX) allgemein definiert. In dieser Formel steht A vorzugsweise für Sauerstoff, Schwefel oder die Gruppierung $-NR^{10}$, worin $R^{10}$ vorzugsweise für Wasserstoff, geradkettiges oder verzweigtes Alkyl mit 1 bis 4 Kohlenstoffatomen sowie für Aryl mit 6 bis 10 Kohlenstoffatomen, insbesondere Phenyl, steht, wobei jeder dieser Arylreste substituiert sein kann durch Hlaogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoff- und bis zu 5 gleichen oder verschiedenen Halogenatomen, wobei als Halogene insbesondere Fluor und Chlor genannt seien. $R^7$ steht vorzugsweise für Wasserstoff oder Methyl. $R^8$ steht in der Formel (IX) vorzugsweise für Wasserstoff, geradkettiges oder verzweigtes Alkyl mit 1 bis 4 Kohlenstoffatomen, Halogenalkyl mit bis zu 3 Kohlenstoff- und bis zu 5 gleichen oder verschiedenen Halogenatomen, wobei als Halogene insbesondere Fluor und Chlor stehen, beispielhaft sei Trifluormethyl genannt, ferner vorzugsweise für Alkenyl und Alkinyl mit 2 bis 4 Kohlenstoffatomen, Cycloalkyl mit 5 bis 7 Kohlenstoffatomen sowie für Halogen, insbesondere Fluor, Chlor oder Brom. $R^8$ steht ferner vorzugsweise für Aryl mit 6 bis 10 Kohlenstoffatomen, insbesondere Phenyl, wobei jeder dieser Arylreste substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. $R^8$ steht ferner vorzugsweise für Aralkyl mit 6 bis 10 Kohlenstoffatomen im Arylteil und 1 bis 4 Kohlenstoffatomen im Alkylteil, insbesondere für Benzyl, wobei jeder dieser Aralkylreste im Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl

mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. Außerdem steht $R^8$ für die Gruppierungen $OR^{11}$, $-SR^{11}$ und $-NR^{10}R^{10}$, worin $R^{10}$ vorzugsweise für diejenigen Reste steht, die oben bereits vorzugsweise für diesen Rest genannt wurden, $R^{11}$ steht in diesen Gruppierungen für Wasserstoff, geradkettiges oder verzweigtes Alkyl mit 1 bis 4 Kohlenstoffatomen, Halogenalkyl mit bis zu 3 Kohlenstoffatomen und bis zu 5 gleichen oder verschiedenen Halogenatomen, wobei als Halogene insbesondere Fluor und Chlor stehen, beispielhaft sei Trifluormethyl genannt, ferner vorzugsweise für Alkenyl und Alkinyl mit 2 bis 4 Kohlenstoffatomen, Cycloalkyl mit 5 bis 7 Kohlenstoffatomen sowie für Aralkyl mit 6 bis 10 Kohlenstoffatomen im Arylteil und 1 bis 4 Kohlenstoffatomen im Alkylteil, insbesondere für Benzyl, wobei jeder dieser Aralkylreste im Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei.

In der Formel (IX) steht $R^5$ vorzugsweise für geradkettiges oder verzweigtes Alkyl mit 1 bis 4 Kohlenstoffatomen. $R^6$ steht in der Formel (X) vorzugsweise für geradkettiges oder verzweigtes Alkyl mit 1 bis 4 Kohlenstoffatomen oder für die Halogene Fluor, Chlor und Brom; der Index p steht für die Zahlen 0, 1 oder 2.

Die bei dem Verfahren (c) als Ausgangsstoffe benötigten N-Azolylalkylaniline der Formel (IX) sind noch nicht bekannt. Man erhält sie, wenn man

d ) Aniline der Formel

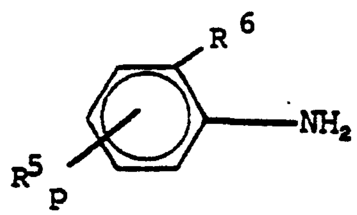

(XI)

in welcher

$R^5$, $R^6$ und p die oben angegebene Bedeutung haben,

mit Azolderivaten der Formel

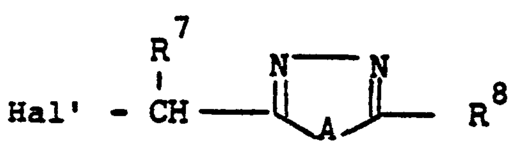

(XII)

in welcher

A, $R^7$ und $R^8$ die oben angegebene Bedeutung haben und

Hal' für Chlor oder Brom steht,

in Gegenwart eines Säurebinders, wie beispielsweise Kalium- oder Natriumcarbonat, und in Gegenwart eines inerten organischen Lösungsmittels, wie beispielsweise Dimethylformamid oder Toluol, bei Temperaturen zwischen 20 und 160°C umsetzt, wobei vorzugsweise ein

Überschuß an Anilin der Formel (XI) eingesetzt wird (vgl. auch Herstellungsbeispiele), oder

e) Hydrazin-Derivate der Formel

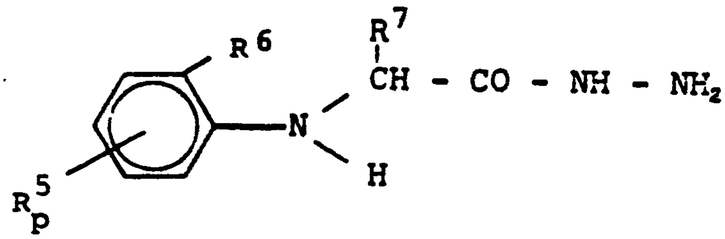

(XIII)

in welcher
$R^5$, $R^6$, $R^7$ und p die oben angegebene Bedeutung haben

mit Isocyanaten bzw. Senfölen der Formel

$$R^{10}-N=C=B \quad (XIV)$$

in welcher
B für Sauerstoff oder Schwefel steht und
$R^{10}$ die oben angegebene Bedeutung hat,

in Gegenwart eines organischen Lösungsmittel, wie beispielsweise eines Alkohols, Äthers oder Kohlenwasserstoffs, bei Temperaturen zwischen 0 und 80°C umsetzt, die entstehenden Verbindungen der Formel

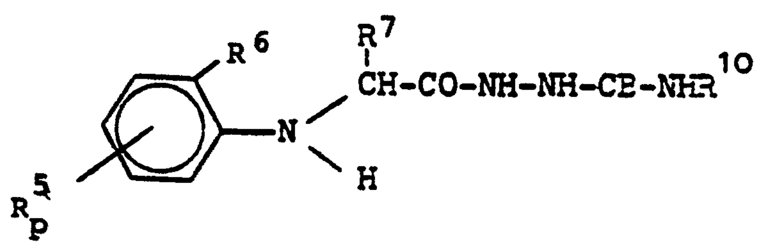

(XV)

in welcher

B,$R^5$,$R^6$,$R^7$ ,$R^{10}$ und p die oben angegebene Bedeutung haben,

in Gegenwart einer starken Base, wie beispielsweise Natron- oder Kalilauge, und in Gegenwart eines Lösungsmittels, wie beispielsweise Aethanol oder Wasser bei Temperaturen zwischen 20 und 100°C cyclisiert und die entstehenden Triazolone bzw. Triazolthione der Formel

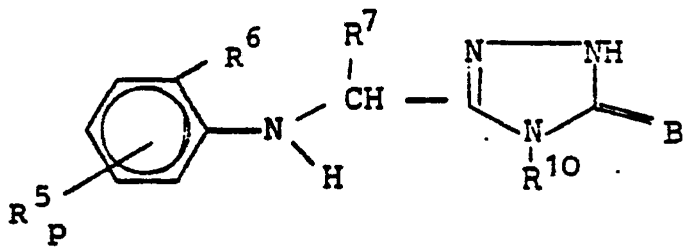

(XVI)

in welcher
B,$R^5$,$R^6$,$R^7$ ,$R^{10}$ und p die oben angegebene Bedeutung haben,

mit Halogeniden der Formel

Hal'-$R^{12}$ (XVII)

in welcher
Hal' für Chlor oder Brom steht und
$R^{12}$ für die Reste des Substituenten $R^{11}$ steht, wobei Wasserstoff ausgenommen ist,

in Gegenwart einer starken Base, wie beispielsweise Natronlauge, und in Gegenwart eines inerten organischen Lösungsmittels, wie beispielsweise Toluol oder Methylenchlorid, bei Temperaturen zwischen 20 und 80°C umsetzt, wobei auch phasentransferkatalysiert und mit anderen Alkylierungsreagenzien, wie beispielsweise Dimethylsulfat, gearbeitet werden kann (vgl. auch Herstellungsbeispiele), oder

f) Hydrazin-Derivate der Formel (XIII) mit Ameisensäure oder Säurechloriden bzw. Säureanhydriden der Formeln

$$R^{13}\text{-CO-Cl} \qquad \text{(XVIIIa)}$$

bzw.

$$(R^{13}\text{-CO-})_2O \qquad \text{(XVIIIb)}$$

in welchen
$R^{13}$ für Alkyl, Halogenalkyl, Alkenyl, Alkinyl, Cycloalkyl, gegebenenfalls substituiertes Aryl oder gegebenenfalls substituiertes Aralkyl steht

in Gegenwart eines inerten organischen Lösungsmittels, wie eines Äthers, Kohlenwasserstoffes oder Halogenkohlenwasserstoffes, bei Temperaturen zwischen 0 und 50°C umsetzt und die entstehenden Verbindungen der Formel

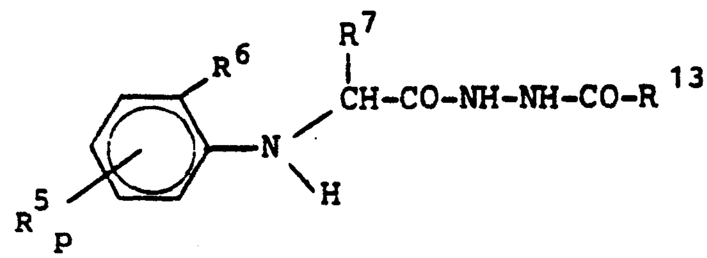

(XIV)

in welcher
$R^5$, $R^6$, $R^7$, $R^{13}$ und p die oben angegebene Bedeutung haben,

entweder mit Diphosphorpentasulfid in an sich bekannter Weise (vgl. Chem.Ber. 32, 797 (1899) und J.prakt. Chemie 69, 145 (1904) zu Thiadiazol-Derivaten cyclisiert, oder ebenfalls in bekannter Weise mit üblichen

wasserabspaltenden Reagenzien zu Oxadiazol-Derivaten umsetzt (vgl. hierzu Elderfield, Heterocyclic Compounds, Vol. 7 (1961) oder

g) Hydrazin-Derivate der Formel (XIII) mit Nitrilen der Formel

$$R^{14}-C\equiv N \qquad (XXI)$$

in welcher

$R^{14}$ für Alkyl, Halogenalkyl oder gegebenenfalls substituiertes Aryl steht

in an sich bekannter Weise zu Triazol-Derivaten umsetzt (vergleiche Chem.Ber. 96, 1064 (1963)), oder

h) Hydrazin-Derivate der Formel (XIII) mit Iminoäthern der Formel

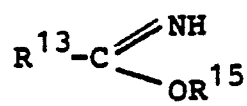 x HCl (XXI)

in welcher

$R^{13}$ für Alkyl, Halogenalkyl, Alkenyl, Alkinyl, Cycloalkyl, gegebenenfalls substituiertes Aryl oder gegebenenfalls substituiertes Aralkyl steht und

$R^{15}$ für Methyl oder Äthyl steht,

in an sich bekannter Weise unter Rückfluß und in Gegenwart eines inerten organischen Lösungsmittels, wie beispielsweise Aethanol, zu Oxadiazol-Derivaten umsetzt, oder

j) die Aniline der Formel (XI) mit Azol-aldehyden der Formel

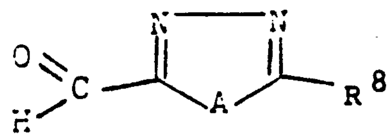

(XXII)

in welcher

$R^8$ die oben angegebene Bedeutung hat,

in Gegenwart eines inerten organischen Lösungsmittels, wie beispielsweise Toluol, bei Temperaturen zwischen 80 und 120°C umsetzt und die entstehenden Verbindungen der Formel

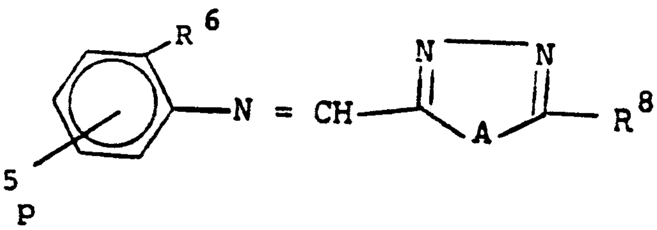

(XXIII)

in welcher

A, $R^5$, $R^6$, $R^8$ und p die oben angegebene Bedeutung haben,

in allgemein bekannter Weise reduziert; z.B. durch Umsetzung mit komplexen Hydriden, wie Natriumborhydrid, gegebenenfalls in Gegenwart eines polaren organischen Lösungsmittels, wie Methanol, bei Temperaturen zwischen 0 und 80°C.

Die bei dem Verfahren d) als Ausgangsstoffe benötigten Verbindungen der Formeln (XI) und (XII) sind bekannt oder lassen sich nach im Prinzip bekannten Verfahren herstellen (vgl. Helv.Chim.Acta 55, 19 9 ff (1972),

Chem.Ber. 32, 797 ff (1899) und Chem.Ber. 96, 1049 ff (1963)).

Die bei dem Verfahren (e) benötigten Ausgangsstoffe der Formel (XIII) sind noch nicht bekannt. Sie lassen sich jedoch nach bekannten Verfahren herstellen, indem man bekannte Ester (vgl. u.a. DT-OS 2 350 944 und 2 513 730) der Formel

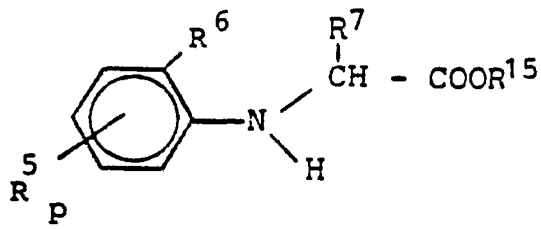

(XXIV)

in welcher
$R^5$, $R^6$, $R^7$ und p die oben angegebene Bedeutung haben und
$R^{15}$ für Methyl oder Aethyl steht,

mit Hydrazinhydrat vorzugsweise in Gegenwart eines organischen Lösungsmittels, wie beispielsweise Äthanol, Dioxan oder Dimethylformamid, bei Temperaturen zwischen 20 und 120°C umsetzt (vgl. auch Herstellungsbeispiele).

Die bei dem Verfahren (e) benötigten Reaktionskomponenten der Formeln (XIV) und (XVII) sind allgemein bekannte Verbindungen der organischen Chemie.

Die bei den Verfahren (f), (g) und (h) als Reaktionskomponenten benötigten Stoffe der Formeln (XVIIIa), (XVIIIb), (XX) und (XXI) sind ebenfalls bekannt.

Die bei dem Verfahren (j) als Reaktionskomponenten zu verwendenden Azol-aldehyde der Formel (XXII) sind ebenfalls bekannt oder lassen sich nach im Prinzip bekannten Verfahren herstellen (vgl. Elderfield, "Heterocyclic Compounds", Vol. 7 (1961) und "Advances in Heterocyclic Chemistry, Vol. 9 (1968)).

Die außerdem für die Umsetzung nach Verfahren (c) als Ausgangsstoffe zu verwendenden Halogenessigsäurechloride bzw. -anhydride sind durch die Formeln (Xa) und (Xb) allgemein definiert. In diesen Formeln steht $R^9$ vorzugsweise für Chlor, Brom und Jod.

Die Halogenessigsäurechloride und -anhydride der Formeln (Xa) und (Xb) sind allgemein bekannte Verbindungen der organischen Chemie.

Als Verdünnungsmittel kommen für die Umsetzung (c) vorzugsweise inerte organische Lösungsmittel in Frage. Hierzu gehören vorzugsweise Ketone, wie Diäthylketon, insbesondere Aceton und Methyläthylketon; Nitrile, wie Propionitril, insbesondere Acetonitril; Aether, wie Tetrahydrofuran oder Dioxan; aliphatische und aromatische Kohlenwasserstoffe, wie Petroläther, Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Das Verfahren (c) kann gegebenenfalls in Gegenwart von Säurebindern (Chlorwasserstoff-Akzeptoren) durchgeführt

werden. Als solche können alle üblichen Säurebindemittel verwendet werden. Hierzu gehören vorzugsweise organische Basen, wie tertiäre Amine, beispielsweise Triäthylamin, oder wie Pyridin; ferner anorganische Basen, wie beispielsweise Alkalihydroxide und Alkalicarbonate.

Die Reaktionstemperaturen können bei der Durchführung des Verfahrens (c) in einem größeren Bereich variiert werden. Im allgemeinen arbeitet man zwischen 0 und 120°C, vorzugsweise zwischen 20 und 100°C.

Bei der Durchführung des Verfahrens (c) setzt man vorzugsweise auf 1 Mol der Verbindung der Formel (IX) bis 1,5 Mol Halogenacetylierungsmittel und 1 bis 1,5 Mol Säurebinder ein. Die Isolierung der Verbindungen der Formel (IV) erfolgt in üblicher Weise.

Die praktische Herstellung von Acetaniliden der Formel (IV) geht aus den folgenden Beispielen hervor.

Beispiel 87

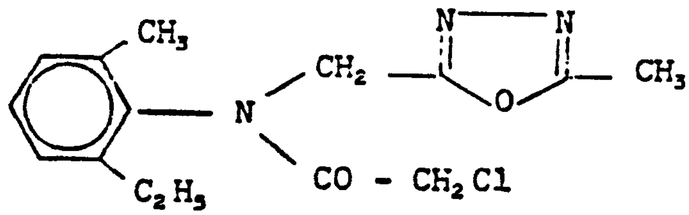

16,3 g (0,07 Mol) 2-Äethyl-6-methyl-N-[(2-methyl-1,3,4-oxadiazol-6-yl)-methyl]-anilin und 6 g (0,076 MOl) wasserfreies Pyridin werden in 100 ml absolutem Tetrahydrofuran unter Rühren zum Sieden erhitzt und tropfenweise mit einer Lösung von 8 g (0,07 Mol) Chloracetylchlorid in 20 ml Tetrahydrofuran versetzt. Nach beendetem Zutropfen läßt man 10 Minuten nachrühren, engt durch Abdestillieren des Lösungsmittels ein und verrührt den Rückstand mit 150 ml Wasser. Das auskristallisierende Reaktionsprodukt wird abgesaugt, mit Wasser gewaschen und getrocknet. Man erhält 18,7 g (87 % der Theorie) beige-farbene Kristalle von 2-Äthyl-6-methyl-N-[(2-methyl-1,3,4-oxadiazol-5-yl)-methyl]-chloracetanilid vom Schmelzpunkt 67 bis 70°C.

Herstellung des Ausgangsproduktes

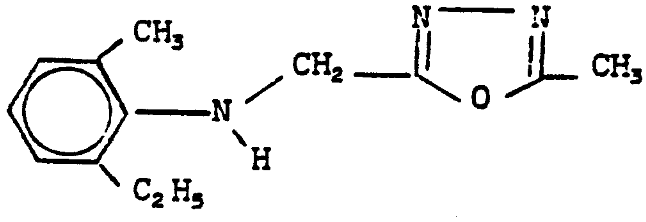

Eine Mischung aus 101,2 g (0,76 Mol) 2-Äthyl-6-methyl-anilin, 40 g (0,3 Mol) 2-Methyl-5-chlormethyl-1,3,4-oxadiazol, 41,4 g (0,3 Mol) gepulvertes Kaliumcarbonat und 76 ml Dimethylformamid wird 5 Stunden unter Rühren auf 100°C erhitzt. Danach wird die Reaktionsmischung filtriert, das Filtrat mit Methylenchlorid verdünnt und mehrmals mit Wasser gewaschen. Die Methylenchloridphase wird über Natriumsulfat getrocknet und im Vakuum durch Abdestillieren des Lösungsmittels eingeengt. Der Rückstand wird im Vakuum destilliert. Man erhält 46,8 g (67,5 % der Theorie) gelbliches Öl von 2-Äthyl-6-methyl-N-[(2-methyl-1,3,4-oxadiazol-5-yl)-methyl]-anilin vom Siedepunkt 140 bis 142°C/0,1 mm mit einer 94 %igen Reinheit (gaschromatographisch bestimmt).

## Beispiel 88

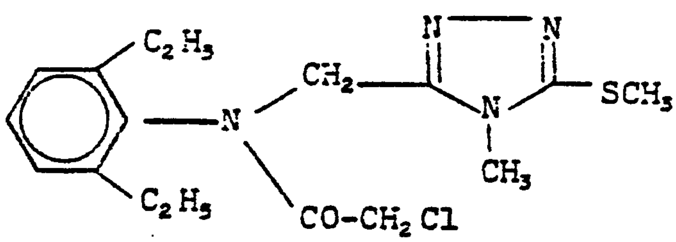

5 g (0,017 Mol) 2,6-Diäthyl-N-[(1-methyl-2-methylthio-1,3,4-triazol-5-yl)-methyl]-anilin und 1,6 g (0,02 Mol) Pyridin werden in 100 ml absolutem Tetrahydrofuran gerührt und bei Raumtemperatur tropfenweise mit 2,3 g (0,02 Mol) Chloracetylchlorid versetzt, wobei die Temperatur auf ca. 30°C ansteigt. Man läßt 2 Stunden rühren, engt teilweise durch Abdestillieren des Lösungsmittels

ein und versetzt mit Wasser. Das auskristallisierende Produkt wird abgesaugt, getrocknet und aus Diisopropyläther/Essigester umkristallisiert. Man erhält 5 g (80 % der Theorie) 2,6-Diäthyl-N-[(1-methyl-2-methylthio-1,3,4-triazol-5-yl)-methyl]-chloracetanilid vom Schmelzpunkt 121 bis 123°C.

Herstellung der Vorstufen

a) 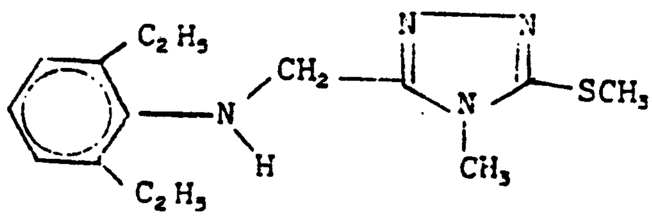

13,9 g (0,05 Mol) 2,6-Diäthyl-N-[(1-methyl-2-thiono-1,3,4-triazol-5-yl)-methyl]-anilin werden bei Raumtemperatur in einem Zweiphasengemisch aus 150 ml Toluol und 40 ml 50 %iger Natronlauge unter Zusatz von 1,5 g Triäthyl-benzylammoniumchlorid (TEBA) als Katalysator schnell gerührt und tropfenweise mit 6,3 g (0,05 Mol) Dimethylsulfat versetzt, wobei die Temperatur auf ca. 35°C ansteigt. Man läßt 5 Stunden rühren, trennt die Toluolphase ab, wäscht sie mehrmals mit Wasser, trocknet über Natriumsulfat und engt durch Abdestillieren des Lösungsmittels ein. Das zurückbleibende Öl wird durch Zusatz von Petroläther zur Kristallisation gebracht. Man erhält nach Umkristallisation aus Petroläther 6,7 g (40 % der Theorie) 2,6-Diäthyl-N-[(1-methyl-2-methylthio-1,3,4-triazol-5-yl)-methyl]-anilin vom Schmelzpunkt 65 bis 67°C.

b) 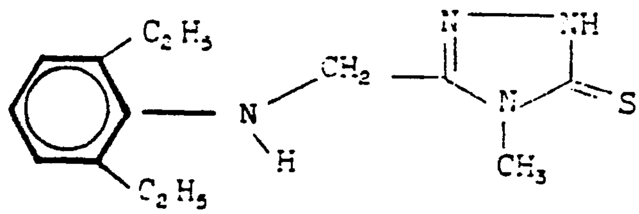

29,6 g (0,1 Mol) 1-Methyl-4-[(2,6-diäthyl-anilino)-acetyl]-thiosemicarbazid werden in 150 ml Äthanol suspendiert und nach Zugabe von 7 g Kaliumhydroxid in 20 ml Wasser 1 Stunde unter Rückfluß erhitzt. Danach wird der größte Teil des Lösungsmittels abdestilliert und der Rückstand mit 250 ml Wasser versetzt. Nach dem Ansäuern mit Eisessig auf pH 5 wird der entstehende Niederschlag abgesaugt und gründlich mit Wasser gewaschen. Nach dem Trocknen erhält man 27 g (97 % der Theorie) 2,6-Diäthyl-N-[(1-methyl-2-thiono-1,3,4-triazol-5-yl)-methyl]-anilin vom Schmelzpunkt 117 bis 121°C.

c) 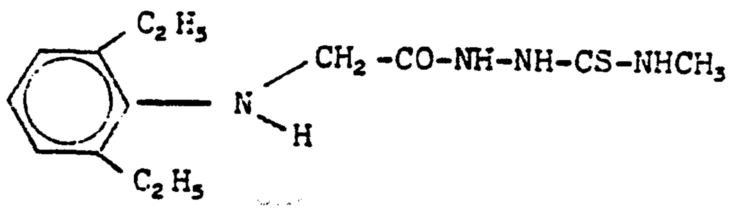

44,2 g (0,2 Mol) 2,6-Diäthyl-anilino-essigsäure-hydrazid und 14,8 g (0,2 Mol) Methylsenföl werden in 250 ml Äthanol gelöst und eine Stunde auf Rückflußtemperatur erhitzt. Nach dem anschließenden Abkühlen auf Raumtemperatur wird der entstandene Niederschlag abgesaugt und zweimal mit je 50 ml Äthanol nachgewaschen. Nach dem Trocknen erhält man 46 g (78 % der Theorie) an 1-Methyl-4/2,6-diäthyl-anilino)-acetyl/-thiosemicarbazid in Form einer farblosen kristallinen Substanz vom Schmelpunkt 166°C.

d) 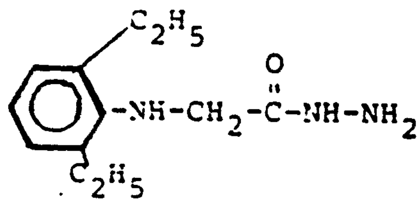

58,7 g (0,25 Mol) 2,6-Diäthyl-anilino-essigsäureäthylester und 25 g Hydrazinhydrat werden in 200 ml Äthanol 24 Stunden stehen gelassen. Danach wird durch Abdestillieren des Lösungsmittels eingeengt und der Rückstand mit Wasser ausgerührt. Nach dem Trocknen erhält man 50,5 g (91 % der Theorie) farblose Kristalle von 2,6-Diäthyl-anilino-essigsäurehydrazid vom Schmelzpunkt 71 bis 73°C.

In entsprechender Weise werden diejenigen Verbindungen erhalten, die in der Tabelle 5 formelmäßig aufgeführt sind.

Tabelle 5

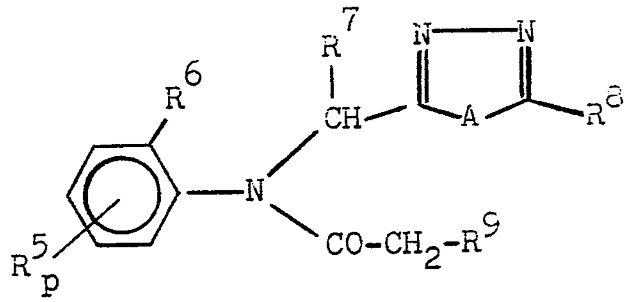

(IV)

| Bsp. Nr. | $R^7$ | $R^8$ | $R^6$ | $R^5_p$ | A | $R^9$ | Schmelzpunkt (°C) |
|---|---|---|---|---|---|---|---|
| 89 | H | $CH_3$ | $C_2H_5$ | 6-$C_2H_5$ | O | Cl | 79-82 |
| 90 | H | $CH_3$ | $CH_3$ | 6-$CH_3$ | O | Cl | 91-93 |
| 91 | H | $CH_3$ | $C(CH_3)_3$ | - | O | Cl | 102-04 |
| 92 | H | $-S-CH_2-CH=CH_2$ | $C_2H_5$ | 6-$C_2H_5$ | 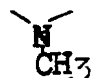 | Cl | 67-70 |
| 93 | H | $-S-CH_2-$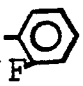 | $CH_3$ | 6-$C_2H_5$ | 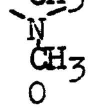 | Cl | 115-20 |
| 94 | H | $C_2H_5$ | $CH_3$ | 6-$C_2H_5$ | O | Cl | 57-59 |
| 95 | H | $C_2H_5$ | $C_2H_5$ | 6-$C_2H_5$ | O | Cl | 43-47 |
| 96 | H | i-$C_3H_7$ | $CH_3$ | 6-$C_2H_5$ | O | Cl | zähfl.Oel |
| 97 | H | $CH_3$ | $CH_3$ | 3-$CH_3$ | 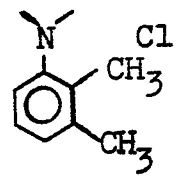 | Cl | glasartig erstarrt |
| 98 | H | $CH_3$ | $C_2H_5$ | 6-$C_2H_5$ | O | Br | 80 |
| 99 | H | $CH_3$ | $CH_3$ | 6-$C_2H_5$ | O | Br | 92-94 |
| 100 | H | $CH_3$ | i-$C_3H_7$ | 6-i-$C_3H_7$ | O | Cl | 135-37 |

Nach einem oder mehreren der in der Anmeldung beschriebenen Verfahren werden die in der nachstehenden Tabelle formelmäßig aufgeführten Ausgangsprodukte erhalten.

Tabelle 6

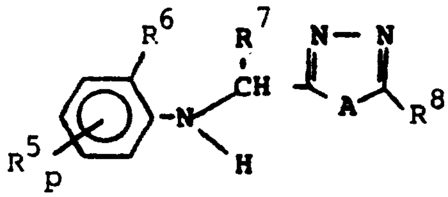

| Beisp. Nr. | $R^7$ | $R^8$ | $R^6$ | $R^5_p$ | A | Schmelzpunkt [°C] bzw. Brechungsindex |
|---|---|---|---|---|---|---|
| IX-1 | H | $CH_3$ | $C_2H_5$ | $6-C_2H_5$ | O | $n_D^{22}$ = 1,540 |
| IX-2 | H | $CH_3$ | $CH_3$ | $6-C_2H_5$ | O | $n_D^{22}$ = 1,547 |
| IX-3 | H | $CH_3$ | $CH_3$ | $6-CH_3$ | O | $n_D^{22}$ = 1,552 |
| IX-4 | H | $CH_3$ | $-(CH_3)_3$ | - | O | 52-55 |
| IX-5 | H | $CH_3$ | $i-C_3H_7$ | $6-i-C_3H_7$ | O | 96-99 |
| IX-6 | H | $C_2H_5$ | $C_2H_5$ | $6-C_2H_5$ | O | $n_D^{22}$ = 1,534 |
| IX-7 | H | $C_2H_5$ | $CH_3$ | $6-C_2H_5$ | O | $n_D^{21}$ = 1,542 |
| IX-8 | H | $i-C_3H_7$ | $CH_3$ | $6-C_2H_5$ | O | $n_D^{21}$ = 1,531 |
| IX-9 | H | $SCH_3$ | $C_2H_5$ | $6-C_2H_5$ | $>N-CH_3$ | 55-57 |
| IX-10 | H | $S-CH_2-CH=CH_2$ | $C_2H_5$ | $6-C_2H_5$ | $>N-CH_3$ | $n_D^{21}$ = 1,577 |
| IX-11 | H | $S-CH_2-$ 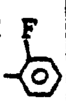 | $CH_3$ | $6-C_2H_5$ | $>N-CH_3$ | zähes Öl |
| IX-12 | H | $CH_3$ | $CH_3$ | $3-CH_3$ | $>N-$ 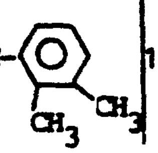 | 142-143 |

Die Acetanilide der Formel (IV) besitzen starke herbizide, insbesondere starke selektive herbizide Eigenschaften. Sie eignen sich daher zur Unkrautbekämpfung. Insbesondere lassen sie sich zur selektiven Unkrautbekämpfung einsetzen.

Das erfindungsgemäß verwendbare Gegenmittel, - das N,N-Diallyl-dichloracetamid der Formel (I) -, eignet sich insbesondere zur Verbesserung der Verträglichkeit von herbizid wirksamen Acetaniliden der Formeln (II), (III) und (IV) bzw. von Säureadditions-Salzen oder Metallsalz-Komplexen von Wirkstoffen der Formel (II) bei wichtigen Kulturpflanzen, wie Mais, Sojabohnen, Baumwolle, Zuckerrüben, Getreide, Reis und Zuckerrohr.

Die erfindungsgemäßen Wirkstoffkombinationen zeigen eine sehr gute Wirkung gegen Unkräuter und Ungräser in zahlreichen Nutzpflanzenkulturen. Sie können daher zur selektiven Unkrautbekämpfung in zahlreichen Nutzpflanzenkulturen verwendet werden. - Unter Unkräutern im weitesten Sinne sind hierbei alle Pflanzen zu verstehen, die an Orten wachsen, wo sie unerwünscht sind.

Die erfindungsgemäßen Wirkstoffkombinationen können zum Beispiel bei folgenden Pflanzen angewendet werden.

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Insbesondere eignen sich die erfindungsgemäßen Wirkstoffkombinationen zur selektiven Unkrautbekämpfung in Mais, Sojabohnen, Baumwolle, Zuckerrüben, Getreide, Reis und Zuckerrohr.

Das erfindungsgemäß verwendbare Gegenmittel der Formel (I) kann gegebenenfalls im Gemisch mit den herbiziden Wirkstoffen, bei denen es eingesetzt wird, in die üblichen Formulierungen übergeführt werden, wie Lösungen, Emulsionen, Spritzpulver, Suspensionen, Pulver, Stäubemittel, Schäume, Pasten, lösliche Pulver, Granulate, Suspensions-Emulsionskonzentrate, Saatgutpuder, Wirkstoff-imprägnierte Natur- und synthetische stoffe, Feinverkapselungen in polymeren Stoffen und in Hüllmassen für Saatgut.

Diese Formulierungen werden in bekannter Weise hergestellt, z.B. durch Vermischen des erfindungsgemäß verwendbaren Gegenmittels gegebenenfalls im Gemisch mit den herbiziden Wirkstoffen, bei denen es eingesetzt wird, mit Streckmitteln, also flüssigen Lösungsmitteln, und/oder festen Trägerstoffen, gegebenenfalls unter Verwendung von oberflächenaktiven Mitteln, also Emulgiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glykol

sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; als feste Trägerstoffe kommen in Frage z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige,

körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent an Gegenmittel bzw. an Gegenmittel und herbizidem Wirkstoff, vorzugsweise zwischen 0,5 und 90 %.

Das erfindungsgemäß verwendbare Gegenmittel kann als solches oder in seinen Formulierungen auch in Mischung mit herbiziden Wirkstoffen eingesetzt werden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Das erfindungsgemäß verwendbare Gegenmittel bzw. Gemische aus erfindungsgemäß verwendbaren Gegenmittel und herbizidem Wirkstoff können als solche, in Form ihrer Formulierungen oder den daraus durch weiteres Verdünnen bereiteten Anwendungsformen, wie gebrauchsfertige Lösungen, Suspensionen, Emulsionen, Pulver und Granulate angewandt werden. Die Anwendung geschieht in üblicher Weise, z.B. durch Gießen, Spritzen, Sprühen, Stäuben, Streuen, Trockenbeizen, Feuchtbeizen, Naßbeizen, Schlämmbeizen oder Inkrustieren.

Das erfindungsgemäß verwendbare Gegenmittel kann nach den für derartige Antidote üblichen Methoden ausgebracht werden. So kann das erfindungsgemäß verwendbare Gegenmittel entweder vor oder nach dem Herbizid ausgebracht oder zusammen mit dem Herbizid appliziert werden. Ferner können Kulturpflanzen durch Saatgutbehandlung mit dem Gegenmittel vor der Saat (Beizung) vor Schäden geschützt werden, wenn das Herbizid vor oder nach der Saat angewendet wird. Eine weitere Einsatzmöglichkeit besteht darin, daß man das Gegenmittel bei der Aussaat in die Saatfurche ausbringt. Wenn es sich bei den Pflanzen um Stecklinge handelt, so können diese vor der Auspflanzung mit dem Gegenmittel behandelt werden.

Beim Einsatz des erfindungsgemäß verwendbaren Gegenmittels kommen die ortsüblichen Aufwandmengen an den jeweiligen Herbiziden zur Anwendung. Die Aufwansmengen an herbizidem Wirkstoff schwanken zwischen 0,1 und 5 kg/ha. Die Aufwandmenge an Gegenmittel ist unabhängig vom Herbizid und der Aufwandmenge des herbiziden Wirkstoffes. Im allgemeinen liegen die Aufwandmengen an erfindungsgemäß verwendbarem Gegenmittel bei Flächenbehandlung zwischen 0,1 und 5 kg/ha, vorzugsweise zwischen 0,2 und 4 kg/ha. Bei der Saatgutbehandlung liegen die Aufwandmengen an erfindungsgemäß verwendbarem Gegenmittel im allgemeinen zwischen 10 und 300 g pro Kilogramm Saatgut, vorzugsweise zwischen 25 und 200 g pro Kilogramm Saatgut.

In den erfindungsgemäßen Wirkstoffkombinationen können die Gewichtsverhältnisse von Gegenmittel zu herbizidem Wirk-

stoff in relativ großen Bereichen schwanken. Im allgemeinen entfallen auf 1 Gewichtsteil an herbizidem Wirkstoff der Formel (II), (III) oder (IV) 0,05 bis 1,0 Gewichtsteile, vorzugsweise 0,1 bis 0,5 Gewichtsteile an Gegenmittel der Formel (I).

Die gute Wirksamkeit des erfindungsgemäß verwendbaren Gegenmittels bzw. der erfindungsgemäßen Wirkstoffkombinationen geht aus dem nachfolgenden Beispiel hervor.

Beispiel A

Pre-emergence-Test

Lösungsmittel: 5 Gew.-Teile Aceton
Emulgator: 1 Gew.-Teil Alkylarylpolyglykoläther

Zur Herstellung einer zweckmäßigen Wirkstoffzubereitung vermischt man 1 Gew.-Teil herbiziden Wirkstoff bzw. eines Gemisches aus Gegenmittel und herbizidem Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Herbizid-Zubereitung bzw. mit der Zubereitung aus Gegenmittel und herbizidem Wirkstoff begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Wirkstoffe, Aufwandmengen und Resultate gehen aus der nachfolgenden Tabelle hervor:

**Tabelle A**

Pre-emergence-Test

| Wirkstoff Herbizid | Wirkstoffaufwand an Herbizid (kg/ha) | Wirkstoffaufwand an N,N-Diallyldichloracetamid (kg/ha) | Schädigung in % bei Mais | Echinochloa | Amaranthus |
|---|---|---|---|---|---|
| 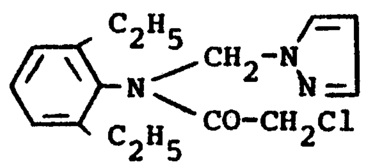 | 3 | - | 70 | 100 | 100 |
| | 3 | 0,5 | 0 | 100 | 100 |
| 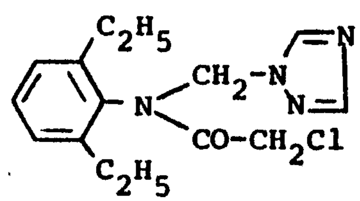 | 3 | - | 30 | 100 | 100 |
| | 3 | 0,5 | 0 | 100 | 100 |
| 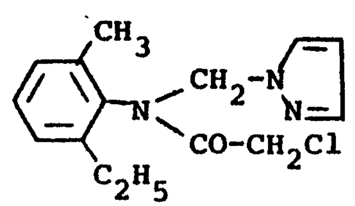 | 3 | - | 90 | 100 | 100 |
| | 3 | 0,5 | 0 | 100 | 100 |

Tabelle A (Fortsetzung)

Pre-emergence-Test

| Wirkstoff Herbizid | Wirkstoffaufwand an Herbizid (kg/ha) | Wirkstoffaufwand an N,N-Diallyl-dichloracetamid (kg/ha) | Schädigung in % bei Mais | Echinochloa | Amaranthus |
|---|---|---|---|---|---|
| 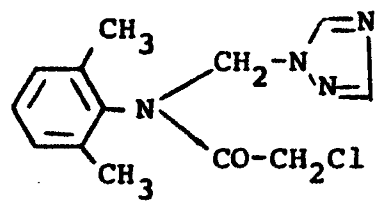 | 3 | - | 70 | 100 | 100 |
| | 3 | 0,5 | 0 | 100 | 100 |
| 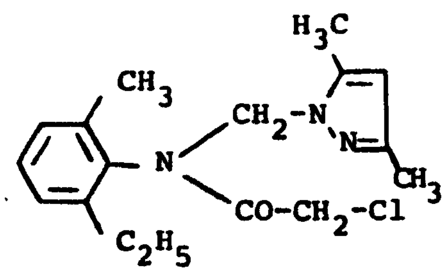 | 3 | - | 90 | 100 | 100 |
| | 3 | 0,5 | 50 | 100 | 100 |

Patentansprüche

1. Verwendung von N,N-Diallyl-dichloracetamid der Formel

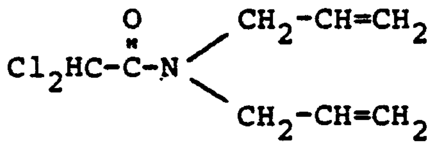

(I)

als Gegenmittel zur Verbesserung der Kulturpflanzen-Verträglichkeit von herbizid wirksamen Acetanilinen

- der Formel

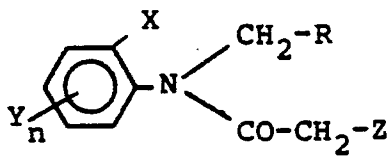

(II)

in welcher

R für einen gegebenenfalls substituierten N-haltigen heterocyclischen Rest stent,

X und Y gleich oder verschieden sind und für Alkyl stehen,

Z für Halogen steht und

n für O, 1 oder 2 steht,

sowie deren herbizid wirksame Säureadditionssalze und Metallsalz-Komplexe

- bzw. der Formel

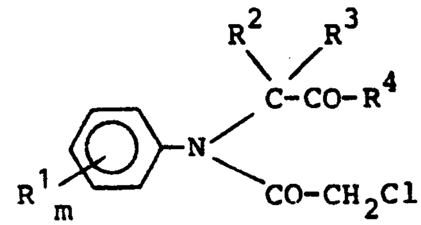

(III)

in welcher

$R^1$ für Alkyl, Halogen, Halogenalkyl, Alkylthio, Alkylsulfonyl, Aminosulfonyl, Cyano oder Nitro steht,

$R^2$ und $R^3$ gleich oder verschieden sind und für Wasserstoff, Alkyl, Halogen, Halogenalkyl oder gegebenenfalls substituiertes Phenyl stehen,

$R^4$ für Alkyl oder gegebenenfalls substituiertes Phenyl steht und

m für ganze Zahlen von 0 bis 5 steht,

- oder der Formel

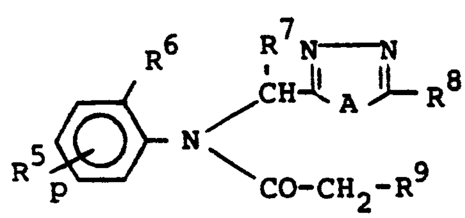

(IV)

in welcher

A für Sauerstoff, Schwefel oder die Gruppierung $NR^{10}$ steht,

$R^7$ für Wasserstoff oder Alkyl steht,

$R^8$ für Wasserstoff, Alkyl, Halogenalkyl, Alkenyl, Alkinyl, Cycloalkyl, Halogen, gegebenenfalls substituiertes Aryl und Aralkyl oder die Gruppierungen $-OR^{11}$, $-SR^{11}$ und $NR^{10}R^{11}$ steht,

$R^{10}$ für Wasserstoff, Alkyl, oder gegebennefalls substituiertes Aryl steht,

$R^{11}$ für Wasserstoff, Alkyl, Halogenalkyl, Alkenyl, Alkinyl, Cycloalkyl oder gegebenenfalls substituiertes Aralkyl steht,

$R^5$ für Alkyl steht,

$R^6$ für Alkyl oder Halogen steht,

$R^9$ für Halogen steht und

p für die Zahlen 0, 1 oder 2 Steht.

2. Verfahren zur Verbesserung der Kulturpflanzen-Verträglichkeit von herbizid wirksamen Acetaniliden der Formeln

(II), (III) und (IV) gemäß Anspruch 1 bzw. von Säureadditions-Salzen oder Metallsalz-Komplexen von Wirkstoffen der Formel (II) gemäß Anspruch 1, dadurch gekennzeichnet, daß man N,N-Diallyl-dichloracetamid der Formel (I) gemäß Anspruch 1 auf die Kulturpflanzen und/oder deren Lebensraum einwirken läßt.

3. Mittel zur selektiven Unkrautbekämpfung in Nutzpflanzenkulturen, gekennzeichnet durch einen Gehalt an einer Wirkstoffkombination bestehend aus
N,N-Diallyl-dichloracetamid der Formel (I) gemäß Anspruch 1
und mindestens einem Acetanilid der Formel (II), (III) oder (IV) gemäß Anspruch 1 bzw. einem Säureadditions-Salz oder Metallsalz-Komplex eines Wirkstoffes der Formel (II) gemäß Anspruch 1.

4. Verfahren zur selektiven Unkrautbekämpfung in Nutzpflanzen, dadurch gekennzeichnet, daß man eine Wirkstoffkombination gemäß Anspruch 3 auf die Unkräuter oder ihren Lebensraum einwirken läßt.

5. Verwendung einer Wirkstoffkombination gemäß Anspruch 3 zur selektiven Unkrautbekämpfung in Nutzpflanzenkulturen.

6. Verfahren zur Herstellung von Mitteln zur selektiven Unkrautbekämpfung in Nutzpflanzenkulturen, dadurch gekennzeichnet, daß man Wirkstoffkombinationen gemäß Anspruch 3 mit Streckmitteln und/oder oberflächenaktiven Mitteln vermischt.